(19) Europäisches Patentamt / European Patent Office / Office européen des brevets

(11) EP 3 394 627 B1

(12) # EUROPEAN PATENT SPECIFICATION

(45) Date of publication and mention of the grant of the patent:
**03.06.2020 Bulletin 2020/23**

(21) Application number: **16819104.7**

(22) Date of filing: **22.12.2016**

(51) Int Cl.:
*G01R 33/56* (2006.01) *A61K 49/10* (2006.01)
*G01R 33/48* (2006.01) *G01R 33/465* (2006.01)
*G01R 33/561* (2006.01)

(86) International application number:
**PCT/EP2016/082446**

(87) International publication number:
**WO 2017/109111 (29.06.2017 Gazette 2017/26)**

(54) **RATIOMETRIC PULSED CEST IMAGING**

RATIOMETRISCH GEPULSTE CEST-BILDGEBUNG

IMAGERIE CEST PULSÉE RATIOMÉTRIQUE

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.12.2015 EP 15202355**

(43) Date of publication of application:
**31.10.2018 Bulletin 2018/44**

(73) Proprietor: **Bracco Imaging S.p.A.**
**20134 Milano (IT)**

(72) Inventors:
- **AIME, Silvio**
  **10126 Torino (IT)**
- **LONGO, Dario, Livio**
  **10126 Torino (IT)**
- **COLOMBO SERRA, Sonia**
  **10010 Colleretto Giacosa (IT)**
- **CONSOLINO, Lorena**
  **10126 Torino (IT)**
- **ARENA, Francesca**
  **10126 Torino (IT)**

(74) Representative: **Ravizza, Claudio**
**Bracco Imaging SpA**
**Intellectual Property**
**Via Caduti di Marcinelle, 13**
**20134 Milano (IT)**

(56) References cited:
- **SILVIO AIME ET AL: "Iopamidol: Exploring the potential use of a well-established x-ray contrast agent for MRI", MAGNETIC RESONANCE IN MEDICINE., vol. 53, no. 4, 1 January 2005 (2005-01-01), pages 830-834, XP055313704, US ISSN: 0740-3194, DOI: 10.1002/mrm.20441 cited in the application**
- **PHILLIP ZHE SUN ET AL: "Quantification of iopamidol multi-site chemical exchange properties for ratiometric chemical exchange saturation transfer (CEST) imaging of pH", PHYSICS IN MEDICINE AND BIOLOGY, INSTITUTE OF PHYSICS PUBLISHING, BRISTOL GB, vol. 59, no. 16, 23 July 2014 (2014-07-23) , pages 4493-4504, XP020268653, ISSN: 0031-9155, DOI: 10.1088/0031-9155/59/16/4493 [retrieved on 2014-07-23]**
- **BENJAMIN SCHMITT ET AL: "Optimization of pulse train presaturation for CEST imaging in clinical scanners", MAGNETIC RESONANCE IN MEDICINE., vol. 65, no. 6, 17 February 2011 (2011-02-17), pages 1620-1629, XP055313973, US ISSN: 0740-3194, DOI: 10.1002/mrm.22750**
- **MOON F.F. ET AL.: "A comparison of iopromide and iopamidol, two acidoCEST MRI contrast agents that measure tumor extracellular pH", PROCEEDINGS OF THE JOINT ANNUAL MEETING ISMRM-ESMRMB, no. 3318, 28 April 2014 (2014-04-28), page 3318, XP040664387,**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 3 394 627 B1

- LONGO D.L. ET AL.: "A Novel CEST-MRI Ratiometric Approach for in vivo pH Imaging", PROCEEDINGS OF THE INTERNATIONAL SOCIETY FOR MAGNETIC RESONANCE IN MEDICINE, 23RD ANNUAL MEETING & EXHIBITION, no. 4666, 15 May 2015 (2015-05-15), XP040670342,
- ZHONGLIANG ZU ET AL: "Imaging amide proton transfer and nuclear overhauser enhancement using chemical exchange rotation transfer (CERT)", MAGNETIC RESONANCE IN MEDICINE., vol. 72, no. 2, 2 December 2013 (2013-12-02), pages 471-476, XP055314015, US ISSN: 0740-3194, DOI: 10.1002/mrm.24953
- ZHONGLIANG ZU ET AL: "Multi-angle ratiometric approach to measure chemical exchange in amide proton transfer imaging", MAGNETIC RESONANCE IN MEDICINE., vol. 68, no. 3, 1 September 2012 (2012-09-01), pages 711-719, XP055314163, US ISSN: 0740-3194, DOI: 10.1002/mrm.23276 cited in the application

## Description

### Field of the invention

The present invention relates to the field of Magnetic Resonance Imaging (MRI) based on pulsed Chemical Exchange-dependent Saturation Transfer (CEST). In particular, it relates to exogenous CEST agent for use in pulsed CEST-MRI to provide Rotation Transfer-based CEST contrast, and to a ratiometric-based CEST-MR procedure applying a pulse train saturation scheme to a single pool of an exogenous CEST agent to obtain concentration-independent CEST contrast.

### State of the art

Chemical Exchange Saturation Transfer (CEST) modality is a recently introduced imaging procedure based on the use of molecules (CEST agents, or CEST systems, as used herein interchangeably) containing one or more exchangeable proton pools.

The CEST imaging technique relies upon a phenomenon which is known in high resolution NMR as double resonance experiment in which the application of a second radio frequency (rf) field centered at, or close to, the chemical shift of the mobile protons, makes possible to saturate their resonance thus creating saturated magnetization that is transferred to the "bulk" water by chemical exchange thus determining a neat reduction of the bulk water signal, which is registered to form an image. This effect is referred to as saturated magnetization transfer, or Saturation Transfer (ST) effect, as used herein interchangeably. The contrast in the resulting MR image is then determined by the extent of the saturated magnetization transfer. Differently from conventional MRI agents, the contrast in the recorded imaging is "frequency-encoded". Indeed, the extent of the reduction of the water signal intensity they promote (determining the strength of the contrast in the registered images) depends on the interplay between physico-chemical properties of the CEST agent and instrumental parameters (typically the applied radio frequency field) (see, for instance, Enzo Terreno, Daniela Delli Castelli and Silvio Aime, Encoding the frequency dependence in MRI contrast media: the emerging class of CEST agents. Contrast Media Mol. Imaging 2010, 5, 78-98).

Basic requisite for a CEST agent is the presence of mobile proton(s) (or exchangeable protons, as herein used interchangeably) having appropriate exchange rate ($k_{ex}$), and suitable chemical shift separation with bulk water protons allowing both exchanging site activation and transfer of the saturation. Roughly, optimal transfer of saturation is when $k_{ex}$ approaches $\Delta v$ ($k_{ex} \cong \Delta v$) where $\Delta v$ is the chemical shift separation in Hz between the two exchanging pools.

CEST contrast agents are mainly grouped in diamagnetic and paramagnetic systems. Suitable examples of low molecular weight diamagnetic CEST agents (DIACEST) were first provided by Balaban in WO 00/66180. Paramagnetic CEST agents (PARACEST), mainly including macrocyclic tetra-amide derivatives of DOTA providing for four magnetically equivalent, or pseudo-equivalent, N-H mobile protons pools, where first reported by Sherry (see, for instance, J. Am. Chem. Soc 2001; 123:1517-1518). The use of iodinated radiographic contrast agents having mobile proton(s) belonging to amide function(s) in magnetization transfer-based MRI techniques was firstly disclosed in US8,211,404.

Details and examples of diamagnetic and paramagnetic CEST agents are e.g. disclosed in Magnetic Resonance Imaging. Chem. Rev. 2010, 110, 2960-3018.

Among CEST agents, a class of particular interest is represented by "responsive" agents, namely contrast agents endowed with at least one exchangeable proton whose saturation transfer capability correlates to a physico-chemical parameter of diagnostic interest of the microenvironment in which the agent distributes. These agents, beside acting as typical CEST agents and providing CEST contrast, are also able to report about changes of the said parameter, typically selected from pH, temperature, metabolites or specific ions concentration, $O_2$ or $CO_2$ partial pressure, proteins or enzymes activity, in the body organ or region in which they distribute, thus acting as useful biomarkers of specific diseases that are strictly related to these changes.

However, as well known in the art, the amount of the saturation transfer ST measured in a CEST procedure depends on the water and CEST agent contents or, in other words, on its local absolute concentration in the concerned organ or tissue of interest. Therefore, the peculiar responsive property exhibited by these responsive agents may, in practice, be properly exploited only when thereof actual concentration is known.

Instead, to be effectively exploitable in *in vivo* determinations, a CEST responsive contrast agent should display its responsiveness in a concentration independent mode.

This task is currently achieved by using CEST agents containing at least two sets of magnetically non-equivalent protons whose ST effect shows a different dependence from the physico-chemical parameter of interest.

In this case, in fact, a standard ratiometric approach (RST) may be exploited, based on the following equations:

$$R_{ST} = \frac{[(M_0 - M_s)/M_s]_{site\,1}}{[(M_0 - M_s)/M_s]_{site\,2}} \qquad (1)$$

pioneered disclosed by Balaban and Ward (for any detail on the above equations see, for instance, Magn. Reson. Med. 2000, 44:799-802) exploiting a comparative ratio between the ST effects induced by the selective irradiation of the two different resonances (or mobile proton pools), respectively identified as site 1 and site 2 in the above equation, that makes the measured ST amount and, in turn, the assessed value of the diagnostic parameter independent on the absolute concentration of the administered CEST probe.

The possible exploitation of the Balaban's approach, however, strictly depends on the development of CEST systems possessing at least two sets of magnetically non-equivalent protons (with different responsiveness to the parameter of interest) having suitable chemical shift separation and appropriate exchange rate ($k_{ex}$) at physiological temperature and pH allowing both exchanging site activation and transfer of the saturation to the bulk water.

In this respect, an important role is exerted by the main $B_0$ field on the chemical shift separation of the two mobile proton resonances; indeed strongest $B_0$ fields result in wider hertz separation, whereas low fields, e.g. between 0.5 and 3T, that are those currently allowed for clinical applications, are detrimental on the said separation.

As a result, the actual exploitability of the Balaban's approach at low $B_0$ field in current clinical use is still challenging, especially with diamagnetic CEST agents of the know art, that typically have a reduced Hz separation between the two available saturable resonances.

An alternative approach comprising obtaining a ratiometric ST effect as a function of the RF irradiation pulse power from CEST agents possessing a single set of mobile protons is disclosed in J. Am. Chem. Soc. 2014, 136, 14333.

By using this approach a concentration independent assessment of the pH was obtained, both *in vitro* and in *in vivo* conditions by using Iobitridol as pH responsive CEST agent.

Both Balaban's and the above approach rely on the exploitation of a continuous wave (CW) saturation scheme, typically using a single long off-resonance RF irradiation having, however, limited applicability on clinical scanners due to their Specific Absorption Rate (SAR) limits.

A pulsed-CEST imaging scheme addressing the SAR and hardware issues by using an irradiation scheme of repetitive short RF pulses is disclosed in Magn Reson Med 2008, 60, 834 and Magn Reson Med 2011, 65, 1620.

The existence of an oscillating CEST contrast component dominated by the rotation effect of the spin magnetization for slow exchanging protons (exchange rate < 100 Hz) of endogenous systems saturated with a pulsed-CEST scheme was observed by Gochberg and colleagues, e.g. disclosed in Magn Reson Med 2011, 66, 1100-1108.

The separation of rotation vs saturation transfer effects in pulsed CEST experiments was dubbed Chemical Exchange Rotation Transfer (CERT).

The existence of a dependence between the rotation CEST contrast obtained by use of pulsed CEST sequence at different irradiation flip angels and the exchange rate $K_{sw}$ of the exchanging mobile proton pool was then established by the same authors, e.g. in Reson Med 2012, 68:711-719, where the ratio of the CEST contrast at multiple flip angle $\theta$ values was used to eliminate the concentration term in the quantitative assessment of $K_{sw}$ values displayed by slow-exchanging endogenous amide and amine protons.

In the cited articles these authors however remark that the observed rotation effect is eliminated under faster exchange conditions compared to "focused endogenous amine exchange" having, as conceded, exchange rate of less than 100 Hz, and that "the oscillating component of the CEST contrast is absent at exchange rates of high as 500 and 1000 Hz (see Magn Reson Med 2011, 66, 1100, e.g. page 1107 and Figure 6).

## Summary of the invention

We have now found that CEST contrast dominated by the rotation effect of the spin magnetization may be obtained by use of exogenous CEST agents.

In particular, we have identified exogenous agents that provide oscillating CEST contrast promoted by rotation effects of the spin magnetization affecting the water signal (or rotation transfer-based CEST contrast, or CERT contrast, as used herein interchangeably) when a pulse train saturation scheme having a frequency centered at (or close to) the chemical shift of a mobile proton of the exogenous agent is applied, acting on said single proton frequency.

Unexpectedly, the identified exogenous agents are able to provide effective rotation transfer-based CEST contrast despite they comprise mobile proton(s) with an exchange rate ($k_{ex}$) with surrounding water protons significantly higher than that recommended in the above quoted prior-art.

Moreover we have found that, despite these exogenous agents may comprise a single pool of mobile protons, they can advantageously be used to set-up ratiometric-based CEST-MRI, e.g. to provide concentration-independent CEST contrast, as well as to set-up CEST-based concentration independent responsiveness.

Indeed, according to the alternative ratiometric-based approach of the invention, an exogenous CEST agents having a single pool of mobile protons may be exploited to obtain ratiometric CEST contrast by comparing CEST effects obtained at different RF irradiation pulse flip angles by using a pulse train saturation scheme. More specifically, the invention concerns the method defined in claim 1.

In particular we have identified an improvement of the conventional ratiometric approach that advantageously allows to obtain a ratiometric CEST contrast independent of the local concentration of the CEST agent by irradiation of a single pool of mobile proton(s) of an exogenous CEST agent with a train of RF pulses at different irradiation flip angles (FA) $\theta$, by keeping constant $B_1$ average power (or $B_{avg\ power}$, defined as the square root of the mean square irradiation field over the entire pulse train period) within a pulsed saturation scheme.

According to this improved approach, a new ratiometric value of the ST effect, otherwise herein called Ratiometric of Pulsed RF flip Angles, or RPA, is obtained as a function of the different RF irradiation pulses flip angles $\theta$, by the comparative ratio between the ST effects induced by the selective irradiation of only one resonance (or mobile proton pool), at (at last two) different RF flip angles.

Interestingly, the exploitation of this new ratiometric approach allows to overcome the prerequisite of the conventionally exploited ratiometric approach using the Balaban's equation (1) requiring, as said, the presence of at least two magnetically non-equivalent mobile protons on the same molecule, thereby resulting in a significant improvement of the general applicability of the ratiometric approach to all the CEST systems, having even a single set of mobile protons.

On the other side, the use of exogenous molecules such as the exogenous CEST agent of the invention, makes it possible to use the method of the invention for obtaining diagnostically relevant information e.g. on the extravasation of the agent (and hence on conditions of the local vascularization) or on the value of extracellular parameters such as pH and temperature, hardly obtainable e.g. by the use of known endogenous amide/amine groups.

The present invention, therefore, generally relies on the use of exogenous agents having a pool of mobile proton(s) (or exogenous CEST agents, as herein used interchangeably) in CEST-MR imaging to generate Chemical Exchange Rotation Transfer-based CEST contrast. Moreover, it relates to a ratiometric-based CEST-MR procedure that comprises using these exogenous agents to set-up CERT-based concentration-independent CEST MR imaging, and as responsive agents to set-up CERT-based concentration independent responsiveness.

In particular, in one aspect the invention relates to the use of exogenous CEST agents comprising a pool of mobile proton(s) in setting a CERT-based CEST-MR imaging procedure that comprises applying a pulse train saturation scheme to a single proton frequency of the agent to generate CERT-based CEST contrast.

In another aspect the invention relates to a CERT-based CEST-MR procedure that comprises applying a train saturation scheme of Radio Frequency (RF) irradiation pulses with different flip angles to a single proton frequency of an exogenous CEST agent, and detecting CERT-based CEST-signals providing for CERT-based CEST contrast.

Preferably, the pulsed (or CERT-based) CEST-MR procedure is ratiometric-based and comprises calculating a ratiometric value of the Saturation Transfer effect as a function of the different RF irradiation pulses flip angles, (said irradiation pulses) acting on a single proton frequency of the exogenous agent.

In particular, in a further aspect the invention relates to the use of exogenous CEST agents in a pulsed CEST-MR procedure that comprises calculating a ratiometric value of the Saturation Transfer effect as a function of different RF irradiation pulses flip angles, to set-up ratiometric-based CEST-MR imaging, or *in vivo* or *in vitro* (*ex vivo*) concentration independent responsiveness.

In an additional aspect the invention relates to an optimized CEST-based ratiometric approach that comprises calculating a new ratiometric value of the CERT-based Saturation Transfer (ST) effect from a single set of mobile proton(s) of an exogenous CEST agent as a function of the different flip angles of RF irradiation pulses acting on the same proton resonance.

In another aspect the invention relates to a multiple-angle ratiometric-based CEST MR procedure that comprises irradiating a single set of mobile proton(s) of an exogenous CEST agent of the invention by a train of RF pulses at different flip angles and calculating a ratiometric value of the ST effect as a function of the different RF irradiation pulses flip angles to set-up concentration-independent CEST contrast and/or to obtain concentration independent *in vivo* or *in vitro* (*ex vivo*) maps of a physiological parameter of diagnostic interest in a human or animal and, preferably, mammalian body organ, fluid or tissue.

More particularly, in a still additional aspect the invention relates to a ratiometric-based CEST-MR procedure that comprises:

a) applying a saturation train of FR irradiation pulses at different flip angles to a single mobile-proton frequency of an exogenous CEST agent,
b) calculating a ratiometric value of the ST effect by comparing ST effects calculated at different RF irradiation pulses flip angles,
c) recording concentration-independent CEST contrast and, optionally,
d) providing concentration independent maps of physical or chemical parameter of diagnostic interest.

These and other objects of the present invention and advantages will be now disclosed in detail in the following description even by means of Figures and Examples.

## Brief Description of Drawings

Figure 1 reports the chemical structure of representative non-ionic radiographic contrast media possessing two not-magnetically equivalent amide resonances with different chemical shift (Iopamidol, at 4.2 and 5.5 ppm) and only one amide proton resonance (Iodixanol, at 4.4 ppm)

Figure 2 reports: panel a) Z-spectrum obtained from a 30 mM solution at 7T, 37°C and pH 6.6 for Iopamidol showing the two distinguishable amide proton resonances at 4.2 and 5.5 ppm, respectively (RF irradiation pulse of 1.5 $\mu$T x 5s); panel b) Z-spectrum obtained from a 30 mM solution at 7T, 37°C and pH 6.6 for Iodixanol showing the unique amide proton resonance at 4.4 ppm (RF irradiation pulse of 3 $\mu$T x 5s).

Figure 3 relates to the test of Example 1 and shows the Chemical exchange rate ($k_{ex}$) for Iodixanol determined in the range of pH 5.5-7.4 from the linewidth of the amide absorbance from1H-NMR spectra at 600 MHZ, T=310K.

Figure 4 reports the plot of CEST contrast as a function of flip angle ($\theta$) for a 40 mM iodixanol solutions having pH values in the range 5.5-7.9 and: (A) $B_{avg\ power}$ 1 $\mu$T and dc 50%; (B) $B_{avg\ power}$ 2 $\mu$T and dc 50% with a total irradiation time of 5 s ($B_0$ = 7T; T=37°C).

Figure 5 relates to the test in vitro of Example 2 and shows: panel a) in vitro MR T2-weighted image of a phantom containing seven different vials with Iodixanol 40 mM solution in PBS at different pH values (5.5 - 6.0 - 6.3 - 6.7 - 7.0 - 7.4 - 7.9); panel b) corresponding ST images obtained upon irradiation with RF train pulses with saturation $B_{avg\ power}$ of 1 $\mu$T and flip angles of 180°; panel c): corresponding ST images obtained upon irradiation with RF train pulses at flip angles of 360° (and same saturation $B_{avg\ power}$ of 1 $\mu$T); panel d) ratiometric RPA map obtained from the ratio of the corresponding ST images of panel b) and c) by using equation (3); panel e) ratiometric RPA pH map overimposed onto $T_2$-weighted image (where the ratiometric RPA was calculated by the new proposed ratiometric approach from the ratio of the corresponding ST images at two different flip angle of 180° and 360° by using equation (3) and the calibration curve of Figure 6b ($B_0$ = 7T, T=310K)).

Figure 6 relates to the test of Example 2 and reports: panel a) plot of the variation of the ST effect as a function of the pH for a Iodixanol 40 mM solution upon irradiation of the unique amide proton pool, at 4.4 ppm with RF irradiation pulse at different flip angles, respectively of 180° and 360° (RF train pulses with $B_{avg\ power}$ of 1.0 $\mu$T, total saturation time of 5s, $B_0$ = 7T and T=37°C); panel b) ratiometric calibration curve showing the dependence of the ratiometric values (RPA) obtained by applying the novel proposed ratiometric procedure to the ST curves of panel a).

Figure 7 reports a plot of the RPA values (from the test of Example 2) as a function of the Iodixanol concentration, from Iodixanol solutions at a variable concentration in the range 10-50 mM ($B_{avg\ power}$ of 1 $\mu$T and dc 50% by rationing 180°/360° $\theta$ values at pH 7.2 (B0 = 7T; T=37°C; total irradiation time of 5 s).

Figure 8 reports a plot of the calculated pH vs experimental pH for 40 mM iodixanol phantoms by using the RPA approach with a $B_{avg\ power}$ = 1 $\mu$T, dc 50%, and rationing 180°/360° $\theta$ values ($R2 = 0.998$, $P<0.001$).

Figure 9 reports: panel a) variation of the ST effect as a function of the pH for Iopamidol (30 mM solution) upon irradiation of the two amide proton pools, at 4.2 and at 5.5 ppm, respectively, for $B_0$ = 7T (37°C, CW irradiation RF pulse of 3 $\mu$T x 5s); panel b) ratiometric calibration curve showing the dependence of the ratiometric values obtained by applying the standard (Balaban's) ratiometric approach to the ST curves of panel a). In the figure, the ratiometric values (RST) are calculated by using equation (1) upon irradiation of the mobile proton pool at 4.2 ppm (site 1) and at 5.5 ppm (site 2), respectively.

Figure 10 reports a plot of CEST contrast as a function of flip angle ($\theta$) for a 30 mM iopamidol solutions with pH ranging from 5.5-7.9, for $B_{avg\ power}$ 1 $\mu$T and dc 50%; total irradiation time of 5 s ($B_0$ = 7T; T=37°C).

Figure 11 reports: panel a) variation of the ST effect with variation of the pH values for a Iopamidol 30 mM solution upon irradiation of the amide proton pool at 4.2 ppm with RF irradiation pulse at different flip angles of 180° and 360° (RF train pulses with $B_{avg\ power}$ of 1.0 $\mu$T, total saturation time of 5s, $B_0$ = 7T and T=37°C); panel b) ratiometric calibration curve showing the dependence of the ratiometric values (RPA) obtained by applying the novel proposed ratiometric procedure to the ST curves of panel a). The ratiometric values RPA of the figure, were calculated by using equation (3) upon irradiation of the mobile proton pool at 4.2 ppm with a train of RF saturation pulses by keeping constant $B_{avg\ power}$ to 1.0 $\mu$T and varying flip angles of 180° ($RF_{FA1}$) and of 360° ($RF_{FA2}$).

## Detailed description of the invention

CEST contrast agents according to the invention comprises exogenous agents having a pool of mobile proton(s) and, hence, even a single pool of mobile proton(s).

In particular, the present invention relates to the use of an exogenous agent comprising a pool of mobile proton(s) in setting a CERT-based CEST-MR procedure where a pulse train saturation scheme is applied to a single frequency of the agent to set-up CERT-based CEST imaging.

In this respect, in the present description, and unless otherwise provided, the expression "exogenous agent" or "Exogenous CEST agent" includes in its meaning any CEST contrast agent or material (having at least one suitable mobile proton) that is active in an individual organism, but that originates outside that organism, namely that is provided or administered to the organism (or is contacted with the organism sample) before imaging.

On the other side, the expression "pool of mobile proton(s)" comprises in its meaning a set consisting of one (namely a single proton) or more mobile protons having the same chemical shift. Exogenous agents according to the invention comprise at least one pool of mobile proton(s) and, hence, one or more mobile protons, having equal (single pool) or different (more pools) chemical shifts.

Suitable examples include diamagnetic agents having a pool of mobile proton(s), including diamagnetic agents with a single pool of mobile proton(s).

Preferably, the mobile proton(s) have exchange rate with surrounding water protons e.g. below 5000 Hz, preferably below 3500, more preferably below 2000 Hz and, most preferably, below 1500 Hz at physiological conditions, namely measured in aqueous solution at a pH about 7, e.g. ranging from about 6.5 to about 7.5, by use of the NMR technique e.g. as disclosed in Magn Reson Med 2011, 65, 202.

Preferred diamagnetic agents comprise mobile proton(s) having moderate to fast exchange rate with surrounding water molecule. In this respect, the phrase "moderate to fast exchange rate" comprises in its meaning an exchange rate ($k_{ex}$) (of a mobile proton or protons pool of the CEST agent of the invention with surrounding water proton(s)) higher than 100 Hz, preferably than 200 Hz, e.g. comprises from about 200 to about 3500 Hz, more preferably from about 200 to about 2000 Hz and, most preferably, from 300 to about 1500 Hz under physiological conditions. Preferably, the mobile proton(s) of these diamagnetic agents further display a chemical shift separation with bulk water protons of at least 1 ppm, preferably of 2 ppm and, most preferably, greater than $k_{ex}$ ($\Delta v \geq k_{ex}$) where $\Delta v$ is the chemical shift separation in Hz between the two exchanging pools.

Preferred exogenous agents for the use of the invention are iodinated contrast agents, (or radiographic contrast agents, as herein used interchangeably), comprising at least one amide mobile proton in their structure.

Suitable examples include iodinated contrast agents of formula (I)

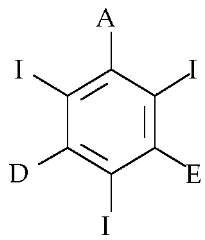

(I)

in which:

A, D, and E the same or different the one another, are selected from the groups of formula $-CON(R)R_1$, $-COOH$, $-CONH_2$ and $-(CH_2)_{0-1}N(R)-COR_2$; in which:

R independently of one another is H or $R_1$;

$R_1$ is a $C_1$-$C_6$ alkyl optionally substituted by one or more hydroxyl (-OH), $C_1$-$C_5$ alkoxy or hydroxyalkoxy groups; or a carbohydrate residue; and

$R_2$ is a $C_1$-$C_6$ alkyl, optionally interrupted by one or more hydroxyl, $C_1$-$C_5$ alkoxy or hydroxyalkoxy groups;

or of formula (II)

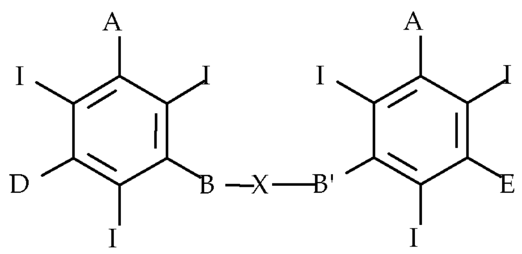

(II)

where:

A, D and E are as above defined,

B and B' the same or different the one another, represent a covalent bond, or a group selected from the groups of formula -CON(R)-, -N(R)CO- and-N($COR_3$)-, in which:

R is as above said;

$R_3$ is H or a $C_1$-$C_3$ alkyl, optionally substituted by one or more hydroxyl groups; and

X is a $C_1$-$C_6$ alkylene, optionally interrupted by a group selected from -O-, -N- and -S-, and optionally substituted by one or more hydroxyl groups,

with the proviso that in the above formulas (I) and (II) at least one R is H.

In this respect, in the present description, and unless otherwise provided, the expression "$C_1$-$C_6$ alkyl" includes in its meanings any linear or branched chain comprising from 1 to 6 carbon atoms such as: methyl, ethyl, propyl, iso-propyl, butyl, iso-butyl, *tert*-butyl, pentyl, hexyl, and the like.

Similarly, the term "$C_1$-$C_3$ alkyl" comprises within its meanings a linear or branched chain comprising from 1 to 3 carbon atoms such as, for instance, methyl, ethyl, propyl and iso-propyl.

By analogy, the expression "$C_1$-$C_6$ alkylene" comprises within its meanings a bivalent, straight or branched, chain derived from any of the corresponding $C_1$-$C_6$ hydrocarbon by removal of two hydrogen atoms from different carbon atoms, including $C_1$-$C_6$ alkylene such as for instance a methylene, ethylene, propylene, iso-propylene, and so on.

Likewise, the term "alkoxy" comprises within its meanings any of the corresponding alkyl-oxy groups such as methoxy, ethoxy, n-propoxy, isopropoxy and the like; the term "hydroxyalkyl" comprises within its meanings any of the corresponding alkyl chain wherein one or more hydrogen atoms are replaced by hydroxyl (OH) groups such as, preferably, hydroxymethyl ($-CH_2OH$), hydroxyethyl ($-CH_2CH_2OH$), hydroxypropyl ($-CH_2CH_2CH_2OH$), dihydroxypropyl, ($CH_2CH_2OHCH_2OH$ and $-CH(CH_2OH)_2$) and the like, and the term "hydroxyalkoxy" comprises within its meanings any of the corresponding $C_1$-$C_5$ alkyl-oxy groups comprising one or more hydroxyls (-OH) in the alkyl chain.

Preferred are iodinated agents of formula (I) in which A, D and E are as reported in Table 1 below together with the name of the intended compound:

Table 1

| Name | A | D | E |
|---|---|---|---|
| Metrizamide | $-CONHCH(CHO)(CHOH)_3CH_2OH$ | -NH-Ac | -N(Me)Ac |
| lopamidol | $-CONHCH(CH_2OH)_2$ | $-NHCOCH(OH)CH_3$ | $-CONHCH(CH_2OH)_2$ |
| lopromide | $-CON(Me)CH_2CH(OH)CH_2OH$ | $-NHCOCH_2OMe$ | $-CONHCH_2CH(OH)CH_2OH$ |
| logulamide | $-CONHCH_2CH(OH)CH_2OH$ | $-NHCOCO(CHOH)_3CH_2OH$ | $-CONHCH_2CH(OH)CH_2OH$ |
| lodamide | -COOH | $-NHCOCH_3$ | $-CH_2NHCOCH_3$ |
| loglucol | -CONHMe | $-NHCO(CHOH)_4CH_2OH$ | $-N(Ac)CH_2CH_2OH$ |
| loglucomide | -CONHMe | $-NHCO(CHOH)_4CH_2OH$ | $-NHCO(CHOH)_4CH_2OH$ |
| loglunide | $-CONHCH_2CH_2OH$ | $-NHCO(CHOH)_4CH_2OH$ | -N(Me)Ac |
| lobitridol | $-CON(Me)CH_2CH(OH)CH_2OH$ | $-NHCOCH(CH_2OH)_2$ | $-CON(Me)CH_2CH(OH)CH_2OH$ |
| locibidol | $-CON(Me)CH_2CH(OH)CH_2OH$ | $-CONHCH_2CH(OH)CH_2OH$ | $-CONH_2$ |

(continued)

| Name | A | D | E |
|---|---|---|---|
| Ioversol | $-CONHCH_2CH(OH)CH_2OH$ | $-N(CH_2CH_2OH)COCH_2OH$ | $-CONHCH_2CH(OH)CH_2OH$ |
| Iotriside | $-CON(Me)CH_2CH(OH)CH_2OH$ | $-CONH_2$ | $-CONHCH_2CH(OH)CH_2OH$ |
| Iomeprol | $-CONHCH_2CH(OH)CH_2OH$ | $-N(CH_3)COCH_2OH$ | $-CONHCH_2CH(OH)CH_2OH$ |
| Iohexol | $-CONHCH_2CH(OH)CH_2OH$ | $-N(COCH_3)CH_2CH(OH)CH_2OH$ | $-CONHCH_2CH(OH)CH_2OH$ |

and the dimeric compounds of the above formula (II) where the meanings for A, B-X-B', D and E are reported in the following Table 2.

Table 2

| Name | A | D = E | B-X-B' |
|---|---|---|---|
| Iofratol | $-CONHCH(CH_2OH)_2$ | $-NHCOCH(OH)CH_3$ | $-CONHCH_2CH(OH)CH_2NHCO-$ |
| Iotasul | $-CON(Me)CH_2CH(OH)CH_2OH$ | $-CON(Me)CH_2CH(OH)CH_2OH$ | $-NHCOCH_2CH_2-S-CH_2CH_2CONH-$ |
| Iodixanol | $-CONHCH_2CH(OH)CH_2OH$ | $-CONHCH_2CH(OH)CH_2OH$ | 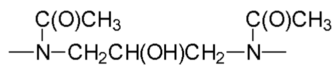 |
| Ioforminol | $-CONHCH_2CH(OH)CH_2OH$ | $-CONHCH_2CH(OH)CH_2OH$ | 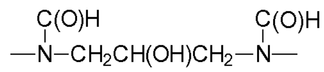 |
| Iotrolan | $-CONHCH(CH_2OH)CH(OH)CH_2OH$ | $-CONHCH(CH_2OH)CH(OH)CH_2OH$ | $-N(CH_3)COCH_2CO(CH_3)N-$ |

More preferred are iodinated contrast agents selected from the group consisting of: Iopamidol, Iodixanol, Iohexol, Ioversol, Iomeprol, Iopromide Iodamide, and possible combinations thereof.

We have now interestingly found that, when a train of RF irradiation pulses (with a frequency tuned to the chemical shift of an exchanging amide proton) is applied to the corresponding amide proton(s) frequency of these radiographic contrast agents, they unexpectedly promotes an efficacy CERT-based CEST contrast despite the amide proton(s) have an exchange rate with water protons well exceeding the 100 Hz recommended in the relevant prior-art.

Indeed, an exchange rate of about 1000 Hz at physiological pH (pH about 7.4) was proven in Example 1 for Iodixanol (Visipaque, GE Healtcare) considered as a representative exogenous CEST agent according to the invention.

Comparable values have also been determined for some additional radiographic contrast agents according to the invention, e.g. provided in Table 3 of the experimental section together with bibliographic references disclosing experimental conditions of performed measures.

An averaged $K_{ex}$ exchange rate value of about 2560 $s^{-1}$ is reported for Iopamidol amide mobile protons (at 7.05 T, 310 K, and pH 7.4) in Magn. Reson. Med. 2005; 53: 830-834.

Moreover, we have interestingly verified that, when the same $B_1$ average power level ($B_{avg\ power}$) is used within a pulsed saturation scheme (or, in other words, when the $B_1$ average power is kept constant during the application of the pulse train saturation sequences at the different flip angles) the CEST effect promoted by these radiographic contrast agents displays a dependence on chemical-physical parameters of diagnostic interest that changes with the flip angle of the RF irradiation pulse.

In particular, we have verified that, when $B_{avg\ power}$ is kept constant, a correlation may be established between the dependency of the CEST effect on a chemical parameter of diagnostic interest, and thus its responsiveness to the same, and the exploited RF irradiation pulse flip angle.

These prompted us to identify a new ratiometric approach, otherwise called multiple-angle ratiometric approach, where a new ratiometric value of the ST effect is obtained by a comparison of the ST effect measured at (at least two) different RF irradiation pulses flip angles.

In particular, we have found that a significant improvement of the conventional ratiometric based approach may be obtained by irradiating a single set of a mobile proton(s) of an exogenous CEST agent according to the invention by a train of RF pulses with different flip angles, and then calculating a new ratiometric value of the ST effect as a function of different RF irradiation pulses flip angles, through a comparison of ST effects obtained at different RF irradiation pulse

flip angles for the same proton resonance.

According to this improved approach, a new ratiometric value of the ST effect, otherwise called Ratiometric of Pulsed RF flip Angles, or RPA, is obtained as a function of the different RF irradiation pulses flip angles (FA), by the comparative ratio between the ST effects induced by the selective irradiation of only one resonance (or mobile proton(s) pool), at (at last two) different RF flip angles, by keeping $B_{avg\ power}$ constant.

Without wishing to include limits on the exploitation of alternative comparative ratios, in one embodiment of the invention the new ratiometric index RPA is calculated by measuring the ratio of the CEST contrast effect promoted at two $\theta$ values according to the following equation:

$$\mathrm{RPA} = \frac{\mathrm{ST}_{\theta 1}}{\mathrm{ST}_{\theta 2}} \quad (2)$$

where $ST_{\theta 1,2}$ represents ST obtained at the selected flip angles $\theta_1$ and $\theta_2$ by keeping $B_{avg\ power}$ constant.

In a preferred embodiment, the value of the new Ratiometric of Pulsed RF flip Angles is determined by means of the following new equation (3)

$$RPA = \frac{[(M_0 - M_s)/M_s)]_{RF_{FA1}}}{[(M_0 - M_s)/M_s)]_{RF_{FA2}}} \qquad (3)$$

where $M_s$ refers to the intensity of the water signal upon irradiation at the frequency corresponding to the mobile protons resonance ($v^{on}$), $M_0$ refers to water proton signal intensity measured upon irradiation at the frequency $v^{off}$ where $v^{off}$ = - $v^{on}$ and $v^{water}$ = 0, $RF_{FA1}$ is the ST effects induced by the selective irradiation of the non-water exchangeable proton resonance at the RF flip angles A1, and $RF_{FA2}$ is the ST effects induced by the selective irradiation of the non-water exchangeable proton resonance at the RF flip angles A2 and RPA is the ratiometric value of the ST effect obtained using the new ratiometric approach of the invention.

From all the foregoing, an object of the instant invention is a CEST-MR procedure that comprises applying a pulse train saturation scheme to a single proton frequency of an exogenous CEST agent comprising a pool of mobile proton(s) to setup *in vivo* or *in vitro* (*ex vivo*) CERT-based CEST-MRI imaging. In particular, the invention relates to a CEST-MR procedure that comprises:

i) applying a pulse saturation scheme by irradiating a single mobile-proton frequency of an exogenous CEST agent in a sample with a train of Radio Frequency (RF) irradiation pulses, at different flip angles and at a constant $B_1$ average power, defined as the square root of the means square $B_1$ irradiation field over the entire period of each pulse train, for inducing a CERT-based Saturation Transfer effect (ST effect); and
ii) detecting CERT-based CEST-signals from said sample.

To this extent, and unless otherwise provided, the term "sample" as used herein refers to an *in vitro* or *ex vivo* biological sample from a human or animal body organ, region, fluid or tissue; or a body organ, region, fluid or tissue of a human or animal patient, that has been previously administered with a suitable amount of the exogenous CEST agent.

In a preferred embodiment said pulsed (or CERT-based) CEST-MR procedure is ratiometric-based, and comprises calculating a ratiometric value of the ST effect as a function of different RF irradiation pulses flip angles.

In particular, in one preferred embodiment the present invention relates to a ratiometric based CEST-MR procedure that comprises applying a train of RF irradiation pulses at different flip angles and constant value of the $B_{avg\ power}$ to a single proton frequency of an exogenous CEST agent and calculating a ratiometric ST effect as a function of the RF irradiation pulses flip angles, i.e. by a comparative ratio between the ST effects measured at the different RF irradiation flip angles.

In a more preferred embodiment the invention relates to a CEST-MR procedure that comprises:

a) applying a saturation train of RF irradiation pulses at different flip angles at a constant value of $B_{avg\ power}$ to a single mobile-proton frequency of an exogenous CEST agent;
b) detecting the CERT-based CEST-signals;
c) calculating a ratiometric value of the ST effect by comparing ST effects measured at different RF irradiation pulses flip angles; and
d) determining a concentration-independent CEST value.

In a preferred embodiment, the step c) comprises calculating the ratiometric value of the ST effect i by exploitation

of the equation (3).

Advantageously, the exploitation of this new ratiometric approach, allows to overcome the prerequisite of the Balaban's approach, i.e. the necessary presence of two non-magnetically equivalent resonances on the same molecules, thereby resulting in a significant improvement of the general applicability of the ratiometric approach to all radiographic contrast agents, and more generally, exogenous agents having only a single pool of exchanging proton(s), although the ratiometric method may be applied as well in the presence of a plurality of exchangeable proton pools.

Moreover, being based on the exploitation of train of repetitive short RF pulses (at different flip angles), the procedure of the invention further allows to overcome long-times requirements and specific SAR drawbacks commonly encountered by using the Continuous-Wave (CW) saturation scheme, and is thus easily translable clinical MRI scanners currently in use.

To this extent, with continuous wave scheme, as used herein we refers to a scheme where a single long (on the order of seconds) off-resonance rectangular radiofrequency irradiation pulse precedes the imaging acquisition.

On the other side, with "repetitive short RF pulses" we refer to a scheme with multiple off-resonance radiofrequency irradiation pulses (on the order of milliseconds and with different shapes: e.g. gaussian, rectangular, sinc.) that precede the readout acquisition.

In one embodiment, said ratiometric-based CEST MR procedure is exploited to provide in *vivo* or *in vitro* (*ex vivo*) images of human or animal body organ, region, fluid or tissue that are unaffected by the local concentration of the CEST agent.

Even more preferably, the ratiometric-based CEST MR procedure of the invention is exploited to provide an *in vitro* (*ex vivo*) or, preferably, *in vivo* assessment or a map of a physical or chemical parameter of diagnostic interest in a human or animal body organ, fluid or tissue that is unaffected by the local concentration of exogenous CEST agent.

In one embodiment, the exogenous CEST agent for use in the pulsed CEST-MR procedure of the invention is a diamagnetic CEST agent.

In a preferred embodiment, the diamagnetic agent is a radiographic contrast agent comprising at least one pool of amide mobile proton(s), e.g. even a single pool of mobile proton(s), that preferably have an exchange rate $K_{ex}$ with surrounding water molecules from 200 to 3500 Hz at physiological conditions.

In one embodiment of the invention, the radiographic contrast agent acting as preferred exogenous CEST agent according to the invention includes only a single pool of mobile protons.

More preferably, the radiographic contrast agent is selected in the group consisting of Iodixanol, Iopamidol, Iopromide, Iohexol, Ioversol, and Iomeprol, and, most preferably, is Iopamidol or Iodixanol.

Accordingly, in a preferred embodiment the present invention relates to a ratiometric-based CEST-MRI procedures that comprises saturating a single amide proton frequency of a radiographic contrast agent with a train of RF irradiation pulses at different flip angles and calculating a ratiometric ST effect as a function of the RF irradiation pulses flip angles to provide concentration-independent *in vivo* or *in vitro* (*ex vivo*) CEST-MRI imaging.

Alternatively, said ratiometric-based CEST MR procedure is employed for the *in vivo* or *in vitro* (*ex vivo*) determination of physical or chemical parameter of diagnostic interest in a human or animal body organ, fluid or tissue that is unaffected by the local concentration of the radiographic contrast agent.

Physical or chemical parameters of diagnostic interest according to the present invention include, for instance, temperature, pH, partial pressure of oxygen ($pO_2$) or carbon dioxide ($pCO_2$), specific ion or metabolite concentration, or specific enzymatic activity, wherein temperature and pH are preferred, and pH is particularly preferred.

In particular, in one preferred embodiment, the invention relates to a ratiometric-based CEST-MR procedure for obtaining concentration-independent images of a human or animal body organ, region, fluid or tissue that comprises:

a) irradiating a single exchanging proton frequency of a radiographic contrast agent with a train of RF irradiation pulses with different flip angles at a constant value of $B_{avg\ power}$;
b) collecting a Z-spectrum;
c) calculating a ratiometric value of the ST effect as a function of the RF irradiation pulses flip angles, preferably by use of the equation 3, e.g. by the a comparative ratio of ST effects obtained at different RF irradiation pulses flip angles acting on the same resonance, and
d) obtaining concentration independent images of said human or animal body organ, region, fluid or tissue,

said determination being performed *in vitro* (ex vivo) e.g. on an in vitro or *ex vivo* sample of a human or animal body organ, region, fluid or tissue previously added with an effective amount of a contrast agent according to the invention, or, preferably, *in vivo,* for obtaining concentration-independent *in vivo* images a human or animal body organ, region, fluid or tissue.

In another preferred embodiment the invention relates to a ratiometric-based CEST-MR procedure according to the invention for determining a physical or chemical parameter or for obtaining a concentration-independent map of a physical or chemical parameter of diagnostic interest in a human or animal body organ, region, fluid or tissue, that

comprises:

A) irradiating a single exchanging proton frequency of a radiographic contrast agent with a train of RF irradiation pulses at different flip angles at a constant value of $B_{avg\ power}$;
B) collecting a Z-spectrum;
C) calculating a ratiometric value of the ST effect as a function of the RF irradiation pulses flip angles, preferably by use of the equation 3 (e.g. by a comparative ratio of ST effects obtained at different RF irradiation pulses flip angles acting on the same resonance); and
D) obtaining, from the calculated ratiometric ST effect a measure of a concentration independent map of physical (or a ratiometric value) of the parameter of interest,

said determination or map being obtained in vitro (ex vivo) e.g. on an in vitro or *ex vivo* sample of a human or animal body organ, region, fluid or tissue previously added with an effective amount of a contrast agent according to the invention or, preferably, *in vivo,* in a human or animal body organ, region, fluid or tissue.

Preferably, the step D) of the above procedure further comprises superimposing the collected map of ratiometric values obtained from step C) of the procedure on previously recorded morphological images of the concerned human or animal body organ, region, fluid or tissue.

To this extent, a skilled practitioner is aware that main imaging steps of the above procedures, including irradiating the mobile proton frequency, collecting the Z-spectrum, calculating a ratiometric ST effect and, by using calibration curves previously performed, obtaining estimates or maps of the desired physical or chemical parameter in a body organ or region, are automatically performed by the MRI scanner, once properly set, in accordance with procedures used in the current diagnostic practice, and by using data processing procedures available to those skilled in the relevant art, for instance from clinical protocols in current use, or provided in the quoted relevant literature.

Accordingly, in one preferred embodiment, the CEST-MRI method of the invention is performed on human or animal bodies that have been previously administered, i.e. suitably pre-administered, with a pharmaceutical preparation comprising a suitable amount of the radiographic contrast agent according to the invention.

In other word, according to a preferred embodiment, the instant invention relates to CEST-MRI method according to the invention for the *in vivo* imaging of a human or animal body organ, region, fluid or tissue or for the in vivo assessment or mapping of a physical or chemical parameter of diagnostic interest in a human or animal body organ, region, fluid or tissue that is carried out on a human or animal body that has been previously administered, namely pre-administered, with a pharmaceutical preparation comprising a suitable amount of a radiographic contrast agent complex compound according to the invention.

With "suitable amount", as used herein, we refers to any amount of a contrast agent of the invention, or pharmaceutical composition thereof, that is sufficient to fulfil its intended diagnostic purpose(s): i.e., for example, to acquire concentration independent contrasted images or providing concentration independent maps of a parameter of interest in a concerned human or animal body organ, region, fluid or tissue, by use of CEST based MRI imaging technique.

Unless otherwise indicated, with "individual patient" or "patient" as used herein we refer to a living human or animal patient, and, preferably a human being undergoing MR diagnostic assessment.

In the method of the invention the saturation transfer step can be carried out according to sequences known in the art, e.g. using repetitive short RF pulses with different shapes (rectangular, gaussian, sinc) in optional combination with different readout schemes, comprising *"gradient or field echo"* and *"spin echo and fast spin echo"* or "echo planar imaging".

In a preferred embodiment, the method of the invention comprises collecting a Z-spectrum covering a range of frequency offset(s) comprising the resonance offset(s) of the mobile proton(s) of the administered CEST agent, or agents mixture, and the water resonance. The Z-spectrum is preferably collected by means of a fast spin echo centric-encoding-based sequence scheme, and optimized through correction on a punctual basis (namely voxel-by-voxel) of $B_0$ inhomogeneities and removal of noisy data, in order to minimize artefacts observed especially when working in vivo conditions (Magn Reson Med 2013, 70, 859).

An optimization of the obtained ST effect is preferably operated on a punctual basis, e.g. by calculating the Saturation Transfer effect (for each of the different flip angle of the RF irradiation pulses) on a voxel-by-voxel basis, i.e. at all the experimental point of each frequency offset, e.g. through an asymmetric interpolation of the Z spectrum by smoothing splines, and translating the interpolated curve in each image pixel in order to fix the minimum of the curve to zero offset value, for instance as disclosed in Development and validation of a smoothing-splines-based correction method for improving the analysis of CEST-MR images, CONTRAST MEDIA & MOLECULAR IMAGING Volume: 3, Issue: 4, Pages: 136-149, 2008; Methods for an improved detection of the MRI-CEST effect, CONTRAST MEDIA & MOLECULAR IMAGING; Volume: 4; Issue: 5; Pages: 237-247; 2009, in order to discriminate (and, hence, remove) noisy or scattered data and to provide, as a result, an increase of the accuracy and sensitivity of the proposed method.

Suitably post processing modalities can also be exploited in combination with the preferred method of the

invention, (e.g. provided in Contrast Media Mol Imaging. 2009, 4, 237-47) further contributing to increase the sensitivity granted by the proposed ratiometric method.

In a particularly preferred embodiment the invention relates to a CEST-MRI method for providing concentration-independent *in vivo* maps of a physical or chemical parameter of diagnostic interest in a human or animal body organ, region, fluid or tissue that comprises:

i) injecting a suitable amount of a radiographic contrast agent into a patient and, optionally, recording anatomical MR images of the body organ, region or tissue of interest;
ii) irradiating a single exchanging proton frequency of the radiographic contrast agent by a pulse train saturation scheme using RF irradiation pulses at different flip angles, at a constant value of $B_{avg\ power}$;
iii) acquiring a Z-spectrum, for instance covering a range of frequency offset comprising the resonance offset of the mobile proton of the administered contrast agent,
iv) interpolating the Z-spectrum on a punctual basis, e.g. by determining the frequency offset of the water signal on a voxel-by-voxel basis, and translating the interpolated curve in each image voxel in order to fix the minimum of the curve to zero offset value;
v) calculating the ST effect in the patient body organ, region or tissue of interest at any desired frequency offset, preferably by using a post processing modality selected from an integral-based, combined, enhanced, and complementary modalities;
vi) calculating the ratiometric value of the ST effect for a single resonance, by the comparison of ST effects at different RF flip angles;
vii) obtaining a map of the parameter of interest by using a previously calculated calibration curve and, optionally
viii) superimposing the resulting ST parametric maps to recorded anatomical MR images, in order to spatially localize the region(s) where the parameter of interest is generated.

Most preferably, in the above CEST-MRI methods of the invention the radiographic contrast agent is Iodixanol.

In particular, in another preferred aspect, the invention relates to the use of Iodixanol as pH responsive agent, to set-up concentration-independent responsiveness, especially to obtain *in vivo* maps of the pH in a human or animal body organ, region, fluid or tissue.

From all the foregoing it stems that, in a particularly preferred aspect, the invention relates to a ratiometric-based CEST-MRI method using a radiographic contrast agent having even a single pool of mobile proton(s) as a responsive agent to provide a concentration independent assessment of a physiological parameter of diagnostic interest though the determination of a ratiometric value of the ST effect based on the exploitation of equations (3).

However, it should be clear to a skilled artisan that encompassed within the scope of the present invention is a ratiometric-based CEST MR procedure that comprises using any exogenous CEST agent according to the invention, namely having at least a pool of mobile proton(s), that preferably are in a moderate to fast exchange regime with water protons.

In the methods of the invention, the radiographic contrast agent acting as CEST agent according to the invention is administered or pre-administered in the form of a suitable pharmaceutical preparation.

In respect, said administration or pre-administration can, for instance, occurs by intravasal injection (for instance intravenous, intraarterial, intraventricular injection, and so on) or intrathecally, intraperitoneally, intralymphaticly, intracavitally, orally or enterally.

Injectable pharmaceutical formulations of exogenous CEST agents according to the invention including, preferably, the radiographic contrast agents according to the formula (I) and (II) having at least one amide proton pool, are typically prepared by dissolving the active ingredient, namely the contrast agent complex, or a pharmaceutically acceptable salt thereof, and the pharmaceutically acceptable excipients in water of suitable purity from the pharmacological point of view. The resulting formulation is suitably sterilized and can be used as such or it can alternatively be lyophilized and reconstituted before the use.

These formulations can be administered in variable concentrations depending on the diagnostic requirements, e.g. ranging from 100 to 450 mg I/mL, although solutions containing lower amounts of the contrast agent can also be used.

Commonly administered CA dose is of from 100 to, more typically, 150 ml of a CA formulation, up to a maximum clinically accepted dose of 1.5 g I/kg of patient BW.

In an especially preferred embodiment, the invention relates to a ratiometric-based CEST procedure for obtaining in vivo maps of the pH in a human or animal body organ, region, fluid or tissue of interest that comprise at least one radiographic contrast agent selected from Iodixanol, Iopamidol, together with suitable excipient, and/ or diluents.

The performance of the provided new ratiometric approach based on the exploiation of the above equation (3) was tested and compared with the classical standard ratiometric modality using the equation (1) by means of in vitro MRI tests reported in detail in the experimental section.

In these tests Iopamidol and Iodixanol were used as non-limiting, representative, examples of exogenous CEST

contrast agents according to the invention.

Iodixanol is a well-known contrast agent used in conventional CT imaging, long time marketed as Visipaque (GE Healthcare). This contrast agent possesses a single proton amide resonance (namely has 4 amide protons having the same resonance in the NMR spectrum, circled in Fig. 1) able to generate a CEST contrast at a specific chemical shift of 4.4 ppm, as shown by the Iodixanol Z-spectrum having only one region of saturation transfer (Figure 2b). Therefore, the Balaban's ratiometric approach, based on the exploitation of at least two magnetically non-equivalent mobile protons, cannot be applied with this compound.

The exchange rate of Iodixanol amide protons in the pH range 5-5 -7.7 was first assessed with the test of Example 1. Obtained results, graphically presented in figure 3, confirm that, at physiological conditions, the measured value of $K_{ex}$ well exceeds 200 Hz, reaching an exchange rate value even higher than 1000 Hz at physiological pH of 7.4.

The responsiveness exhibited by this compound toward pH was then been verified, by means of in vitro MRI experiments reported in detail in the Examples 2 and 3 of the experimental section.

The test was performed by using a phantom containing seven different vials with Iodixanol solutions (40 mM solution in PBS) at different pH values ranging from 5.5 and 7.9. CEST experiments where performed by use of RF train pulses with constant saturation $B_{avg\ power}$ of 1 $\mu$T and different flip angles ($\theta$), respectively of 180° and 360°.

Obtained ST values, shown in Figure 5, panels b) and c), confirm that, when the $B_{1average}$ power level is kept constant in the experiment, the dependence of the saturation transfer on the pH changes with the flip angle ($\theta$) of the RF irradiation pulse applied to the unique amide proton pool of Iodixanol.

A ratiometric RPA map was thus obtained, by using equation (3), from ST images of paned b) and c), shown in the paned d) of the figure, leading to obtain a concentration independent assessment of the pH, provided in the panel e) of the figure, where the obtained pH map is overimposed onto $T_2$-weighted image.

Interestingly, the use of Iodixanol with the ratiometric method of the invention enabled the determination of concentration independent measures of the pH in the physiological range of 6-7.9 with a good accuracy, that have proven to be highly correlated with corresponding pH values measured by the pH-meter (Pearson's r=0.993, P<0.0001), as shown in figure 8.

Moreover, the proposed ratiometric method has proven to display a robust independence from the concentration, as confirmed by the results of the test of Example 2, graphically presented in figure 7.

The performance provided by the new ratiometric approach and its sensitivity toward the pH was then tested by comparison with corresponding results obtained by using the standard ratiometric modality currently in use.

In particular a comparison was performed between the responsiveness to the pH measured by using the ratiometric approach of the invention exploiting a train of RF irradiation pulses at different flip angles and the equation (3), and the standard approach exploiting a Continuous Waves (CW) irradiation scheme and a ratiometric approach based on the Balaban's equation (1).

Comparative tests, described in detail in the Examples 4 and 5 of the experimental section, were performed by using Iopamidol as a CEST agent according to the invention that comprises two pools of mobile protons, respectively at 4.2 and 5.5 ppm (Figures 1 and 2) enabling the exploitation of the conventional Balaban's approach.

ST values and ratiometric ST effect as a function of the pH obtained by use of the equation (1) according to the standard ratiometric approach are graphically provided in figure 9.

Corresponding ST effect and ratiometric ST curves as a function of the pH obtained by application of a pulsed irradiation scheme for the amide proton pool at 4.2 ppm ($B_{avg\ power}$ = 1 $\mu$T x 5s, T=310K) at different irradiation flip angles (180 and 360°), according to the procedure of the invention are provided in Figure 11.

Interestingly, obtained results (in Figure 9b and 11 b) are fully comparable, and allow toconfirm that the pH responsiveness obtained with the method of the invention is consistent and comparable with the pH responsiveness obtained with the standard ratiometric approach.

The sensitivity granted by the ratiometric method of the invention to report on a particular parameter of diagnostic interest, (e.g. the physiological pH) was then assessed as the difference (or delta) between the lower and the higher ratiometric value (of the parameter of interest, e.g. the physiological pH) measured inside the range of examined values.

Unexpectedly, a pH sensitivity with a delta of 1.4 unit was obtained by use of the ratiometric approach of the invention, fully comparable with the corresponding value obtained with the standard ratiometric approach at 7T (sensitivity = 1.4 units) (Figures 9b and 11b).

This result besides being particularly good, is unexpected, especially in light of the fact that the proposed procedure is based on the exploitation of short pulsed sequences that avoid SAR effects and are less uncomfortable for the patient but that, for contrast, commonly result in a reduction of the enabled sensitivity.

## EXPERIMENTAL PART

### Abbreviation

The following common abbreviations are used throughout the specification and the following experimental section; several others are not herewith reported as being conventionally adopted.

| | |
|---|---|
| MRI = | Magnetic Resonance Imaging |
| CEST= | Chemical Exchange Saturation Transfer |
| CERT = | Chemical Exchange Rotation Transfer |
| DIACEST: | Diamagnetic CEST agents |
| PARACEST = | Paramagnetic CEST agents |
| MT = | Magnetization Transfer |
| ST = | Saturation Transfer |
| RF = | Radio Frequency |
| CW = | Continuous Wave saturation scheme |
| FA = | flip angle |
| dc = | duty cycle, or RF pulse duration |
| $B_0$ = | main magnetic field |
| $B_1$ = | RF saturation power |
| $B_{avg\ power}$ = | is the field strength of a continuous wave irradiation with the same average power as the pulsed-CEST (according to Magn. Reson Med 2012, 68:711-719) |
| RARE = | Rapid Acquisition with Relaxation Enhancement |
| RPA = | Ratiometric of Pulsed flip Angles measured according to the Equation (3) |
| RST = | Ratiometric Saturation Transfer measured according to the Equation (1) |
| $RF_{FA}$ = | RadioFrequency irradiation at a specific Flip Angle |
| ROI = | Region Of Interest |
| PBS = | Phosphate Buffer Solution |
| SAR = | Specific Absorption Rate |

### MRI Experiment

MRI acquisitions

MR images were acquired on a Bruker Avance 300 spectrometer operating at 7 T and equipped with a microimaging probe with a transmiter/receiver resonator (diameter of 30 mm) used for in vitro and in vivo experiments, respectively. Z-spectra were acquired in the frequency offset range from -10 to +10 ppm, with step size of 0.1 ppm, using a Spin Echo RARE sequence (typical setting TR/TE/NEX/RARE factor = 8000 ms/8 ms/3/64) preceded by a CW saturation scheme (single block pulse, duration 5 s) or, according to the new procedure of the invention, by using a train of Gaussian pulses by keeping constant the average $B_1$ power (0.5 - 3.0 $\mu$T) and changing the flip angle of the Gaussian irradiation pulses (range 45-900°). Each irradiation pulse had duration $\tau_P$, flip angle $\theta$, interpulse delay $\tau_D$ and the pulse train repetition (PTR) is given by $\tau_P + \tau_D$.

For example, a pulsed-CEST sequence with a $B_{avg\ power}$ of 1 $\mu$T, irradiation FA of 180° and dc of 50% has a $\tau_P$ of 10.8 ms, a $\tau_D$ of 10.8 ms, a pulse train repetition of 21.6 ms, and 232 pulses for a total irradiation time of 5s. $B_{avg\ power}$ levels were set to be 0.5, 1 and 2 $\mu$T with different values of duty cycle (dc) of 50% and 30%. To test the predicted angular dependence, 15 values between 45 and 900° were acquired for each $B_{avg\ power}$ level and dc conditions. For pulsed-CEST imaging, $B_{avg\ power}$ can be calculated by using the following equation (4)

$$B_{avg\ power} = \sqrt{\frac{1}{PTR}\int_0^{PTR} B_1^2 dt} = \sqrt{\frac{p_2}{dc}} \cdot \frac{\pi\theta}{180 \cdot \gamma \cdot p_1 \cdot PTR} \qquad (4)$$

where $B_{avg\ power}$ is the field strength of a continuous wave irradiation with the same average power as the pulsed-CEST. p1 is the ratio of the average amplitude to the maximum amplitude of the irradiation pulse, and p2 is the ration of the average of the square of the amplitude to the square of the maximum amplitude of the irradiation pulse and $\gamma$ is the gyromagnetic ratio of the proton (with units rad(s T). For the Gaussian pulse used in our experiments p1 is equal to 0.418 and p2 equal 0.299, respectively.

Images were acquired with a 64x64 acquisition matrix with a slice thickness of 2 mm and a field of view (FOV) of 30x30 mm. The experiments were performed at 37 °C.

Image and data analysis

Z-spectra raw data were exported from the MRI workstation and automatically elaborated by means of a home-written software running in MATLAB (The Mathworks, Inc., Natick, MA, USA). The analysis consisted of several steps including image segmentation, global ROI analysis, local voxel-by-voxel analysis. In particular, after the automatic image segmentation of the morphological image, a Region Of Interest (ROI) was manually selected, in each morphological image, commonly including tumor area, blood vessels or edema regions, in which ROI features such as the bulk water resonance frequency and the ST response are investigated. Z-spectra were built by sampling the signal with a 0.1 ppm sampling step, more typically used for the irradiation frequency values close to the bulk water resonance. Collected Z-spectra were successively analyzed by using smoothing splines, to obtain the ratiometric value of the ST effect in the identified ROIs by using a software compiled in MATLAB platform, for instance as disclosed in CONTRAST MEDIA & MOLECULAR IMAGING Volume: 3, Issue: 4, Pages: 136-149, 2008, and in CONTRAST MEDIA & MOLECULAR IMAGING; Volume: 4; Issue: 5; Pages: 237-247; 2009.

In particular, on the basis of the global analysis of the selected ROI, the segmented image was locally investigated by calculating the ST at any desired frequency offset. A local analysis was performed, on a voxel-by-voxel basis, accounting for the different physico-chemical properties in each single voxel, by interpolating samples coming from each voxel of the segmented image, in order to obtain the local Z-spectrum. The interpolation was performed based on smoothing splines, by using a smoothing factor of 0.9, allowing to identify and subtract voxel with low signal-to-noise ratio.

The right position of the bulk water signal was assessed on a voxel basis, leading to obtain a "zero offset" map of the voxel-by-voxel distribution of the bulk water resonance in the segmented image. The locally corrected ST analysis was then carried out by shifting the interpolated Z-spectrum in each voxel in order to fix the bulk water resonance to zero offset. Then, a correct intra-voxel ST was calculated on the basis of the shifted interpolated curve.

Depending on the exploited ratiometric approach (and therefore to the corresponding used equations (1) and (3)), the single voxel ST effects at different resonances, or at different RF irradiation flip angles, were ratioed to obtain the resulting ratiometric value. By using a calibration curve previously performed in in vitro experiments, the corresponding pH map was calculated and overimposed on the corresponding anatomical image.

**Example 1**

Test in vitro

Assessment of the Chemical exchange rate of Iodixanol in the pH range 5.5-7.9.

High-resolution 1H-NMR spectra were recorded on a Bruker Avance 600 spectrometer (Bruker BioSpin Rheinstetten, Germany) operating at 14 T (corresponding to a proton Larmor frequency of 600.13 MHz) equipped with a Z-gradient 5 mm inverse probe. Sample temperature control was monitored within +/- 0.1 K by means of the BTO2000 variable temperature unit. 1H NMR spectra to measure the linewidth of exchangeable protons were obtained by means of a pulse sequence using water suppression by excitation sculpting with gradients (T. L. Hwang, A. J. Shaka Water Suppression That Works. Excitation Sculpting Using Arbitrary Wave-Forms and Pulsed-Field Gradients, J. Magn. Reson. Ser. A, 1995, 112(3), 275-279). The carrier frequency was carefully set on resonance to the water signal. Simple "excite-detect" acquisitions (typical parameters: 16 ppm spectral window, 32k complex data points, 3s relaxation delay, and 64 averaged FIDs) were carried out to measure the linewidths of the exchangeable amide protons at 310 K. In vitro studies were performed by preparing 30 mM solutions of Iodixanol in aqueous phosphate buffer solution (10 mM), with the pH adjusted within the 5.5-7.4 range by addition of aliquots of HCl/NaOH. Capillaries containing the solutions were transferred into a 5 mm NMR tube filled with $D_2O$ for field-frequency lock. Exchange rates as a function of pH are shown in Figure 3.

Exchange rate values analogously assessed in the pH range of from 5.4 to 7.9 for some radiographic contrast agents according to the invention are provided in the following Table 3.

Details concerning the frequency of exchanging amide protons and experimental conditions are provided in the quoted reference.

Table 3

| $K_{ex}$ ($s^{-1}$), 310 K | | | | |
|---|---|---|---|---|
| pH | Iomeprol* | Iohexol* | Ioversol* | Iodixanol* |
| 5.5 | 30 | 22 | 22 | 32 |
| 6.0 | 88 | 69 | 66 | 51 |
| 6.3 | 164 | 139 | 118 | 80 |
| 6.7 | 430 | 372 | 292 | 179 |
| 7.0 | 794 | 923 | 913 | 476 |
| 7.4 | 1831 | 1609 | 1628 | 1213 |
| 7.9 | 4389 | 4056 | 3660 | 1332 |
| *Investigative Radiology, Vol.00:00-00, Published Ahead-of-Print 12 Oct. 2015 - DOI: 10.1097/RLI.0000000000000217 | | | | |

**Example 2**

Test in vitro

Responsiveness of Iodixanol towards pH at 7T with the new proposed ratiometric approach.

The responsive properties of Iodixanol towards pH has been investigated in vitro by using a phantom containing seven capillaries filled with solutions of Iodixanol at 40 mM and different pH, ranging from 5.5 to 7.9. CEST MRI experiments have been performed at 37°C and at $B_0$ = 7T. MR images of the phantom itself with the different pH values is shown in Figure 5a, while corresponding ST maps obtained upon irradiation of the amide resonance at 4.4 ppm with a gaussian train saturation scheme (keeping constant the $B_1$ average power at 1 $\mu$T) at the two different flip angles of 180° and 360° are shown in Figure 5b and 5c, respectively. For each capillary mean ST values were calculated from the ST images and plotted as a function of pH values and shown in Figure 6a. In particular, the Figure 4a shows the different ST profiles obtained for different flip angles, confirming the dependence from pH of the saturation transfer displayed by the single amide proton pool, enabling therefore the use of the proposed ratiometric RF flip angles mismatched approach. Calibration curves have been then performed, by ratioing the variation of the ST effects with variation of the pH exploiting equations (3), consenting to obtain the ratiometric curves of Figure 6b between RF irradiation pulses of 180°/360°. A pH map was then calculated by applying the ratiometric method of equation (3) to the ST images of figure 5c-d. More particularly, pixel- by-pixel the ratio of the ST effects measured, respectively, at 180° and at 360° is converted in the corresponding pH value by using the calibration curve of Figure 6b. A pH map is thus obtained and superimposed on anatomical images formerly obtained, shown in Figure 5e.
The pH sensitivity of the ratiometric approach has been calculated as the difference in the ratiometric values between the two pH values of 6.0 and 7.4, range that encompasses the physiological pH range. For the proposed flip angle based pulsed ratiometric approach, when employing a $B_1$ average power of 1 $\mu$T, the pH sensitivity is equal to 1.4, as calculated from Figure 6b.

**Example 3**

Test in vitro

Assessment of the accuracy and concentration independence of the pH value obtained using the proposed ratiometric approach.

The pH responsive properties of Iodixanol at 7T have been investigated in vitro by using a phantom with five capillaries comprising Iodixanol solutions at pH 7.2 and concentrations ranging from 10 to 50 mM. CEST MRI experiments have been performed at 37°C and $B_0$ = 7T, with a train pulse saturation scheme ($B_1$ average power of 1 $\mu$T) with two irradiation flip angles of 180° and 360° for a total saturation time of 5s. Upon irradiation of the phantom, a ST effect was measured for each flip angle and corresponding ratiometric RPA values obtained using equation (3) as a function of iodixanol concentrations. Obtained results, schematized in Figure 7, confirm the robust concentration independence of

the ST effect obtained by ratioing the ST contrast measured at different flip angles, according to the new ratiometric approach set-up by the invention.

The accuracy of the pH values obtained by use of the ratiometric approach of the invention within the physiological pH range of 6-7.9 was then tested, by comparison of measured ratiometric pH values with corresponding pH values measured by the pH-meter.

A high correlation (Pearson's r=0.993, P<0.0001) between ratiometric and corresponding pH-meter values was thus verified, provided in Figure 8, confirming the good pH accuracy ensured by the provided ratiometric approach in the tested pH range.

**Example 4**

Test in vitro

Assessment of the responsiveness of Iopamidol towards pH with the standard ratiometric approach ($B_0$ = 7T).

The responsive properties of Iopamidol towards pH has been investigated in vitro by using a phantom containing seven capillaries filled with solutions of Iopamidol at 30 mM and different pH, ranging from 5.5 to 7.4. 1H-NMR spectra have been acquired

CEST MRI experiments have been performed at 37°C and at B0 = 7T upon irradiation of the amide resonances at 4.2 ppm and at 5.5 ppm with CW saturation pulse ($B_{avg\ power}$ 3 $\mu$T, irradiation time 5s). For each capillary mean ST values were calculated from the ST images and plotted as a function of pH values and shown in Figure 9a. In particular, the Figure 9a shows the ST profiles obtained for the two different amide resonances, confirming the dependence from pH of the saturation transfer displayed by the two amide proton pools, enabling therefore the use of the standard ratiometric approach. Calibration curves have been then performed, by ratioing the variation of the ST effects with variation of the pH exploiting equations (1), consenting to obtain the ratiometric curves of Figure 9b.
For the standard ratiometric approach, the pH sensitivity for Iopamidol is equal to 1.4, as calculated from the Figure 9b.

**Example 5**

Test in vitro

Assessment of the Responsiveness of Iopamidol towards pH with the new proposed ratiometric approach ($B_0$ = 7T).

The responsive properties of Iopamidol towards pH has been investigated in vitro by using a phantom containing seven capillaries filled with solutions of Iopamidol at 30 mM and different pH, ranging from 5.5 to 7.9. CEST MRI experiments have been performed at 37°C and at B0 = 7T. For each capillary mean ST values were calculated from the ST images and plotted as a function of pH values and shown in Figure 10. In particular, the Figure 10 shows the different ST profiles obtained for different flip angles, confirming the dependence from pH of the saturation transfer displayed by the amide proton pool at 4.2 ppm, enabling therefore the use of the proposed ratiometric RF flip angles mismatched approach. ST curves as a function of pH values acquired upon train pulses irradiation at two flip angles of 180° and 360° are shown in Figure 11a. Calibration curves have been then performed, by ratioing the variation of the ST effects with variation of the pH exploiting equations (3), allowing to obtain the ratiometric curves of Figure 11b between RF irradiation pulses of 180°/360°.

**Claims**

1. A CEST-MR procedure that comprises:

   i) applying a pulse saturation scheme by irradiating a single mobile-proton frequency of an exogenous CEST agent in a sample with a train of Radio Frequency (RF) irradiation pulses, at different flip angles and at a constant $B_1$ average power, defined as the square root of the mean square $B_1$ irradiation field over the entire period of each pulse train, for inducing a chemical exchange rotation transfer (CERT)-based Saturation Transfer effect (ST effect); and
   ii) detecting CERT-based CEST-signals from said sample.

2. The CEST-MR procedure of claim 1 wherein said procedure is ratiometric based.

**3.** The CEST-MR procedure of claim 2 that comprises calculating a ratiometric value of the ST effect as a function of the different RF irradiation pulses flip angles.

**4.** The CEST-MR procedure of any one of claims 2 or 3 that comprises:

a) calculating a ratiometric value of the ST effect by comparing ST effect values measured at different RF irradiation pulses flip angles;
b) determining a concentration-independent CEST value.

**5.** The CEST-MRI procedure of claim 4 wherein the step a) comprises calculating a Ratiometric of Pulsed RF flip Angles by exploitation of the equation (3)

$$RPA = \frac{\left[\left(M_0 - M_s\right)/M_s\right]_{RF_{FA1}}}{\left[\left(M_0 - M_s\right)/M_s\right]_{RF_{FA2}}} \qquad (3)\ .$$

**6.** The CEST-MR procedure of claim 4 wherein the concentration independent CEST value is used to provide a concentration independent CEST image.

**7.** The CEST-MR procedure of claim 4 wherein the concentration independent CEST value is used to provide a concentration independent parametric image.

**8.** The CEST-MR procedure of any one of claims 1-7 wherein the exogenous CEST agent is diamagnetic.

**9.** The CEST-MR procedure of claim 8 wherein the diamagnetic agent is a radiographic contrast agent having at least one amide proton.

**10.** The CEST-MR procedure of claim 9 wherein the radiographic contrast agent is a iodinated contrast agent of formula (I)

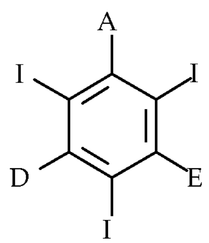

(I)

in which:

A, D, and E the same or different the one another, are selected from the groups of formula $-CON(R)R_1$, $-COOH$, $-CONH_2$ and $-(CH_2)_{0-1}N(R)-COR_2$; in which:

R independently of one another is H or $R_1$;
$R_1$ is a $C_1$-$C_6$ alkyl optionally substituted by one or more hydroxyls, $C_1$-$C_5$ alkoxy, $C_1$-$C_5$ hydroxyalkoxy, or a carbohydrate residue; and
$R_2$ is a $C_1$-$C_6$ alkyl optionally interrupted by one or more hydroxyl, $C_1$-$C_5$ alkoxy, or $C_1$-$C_5$ hydroxyalkoxy group;

or of formula (II)

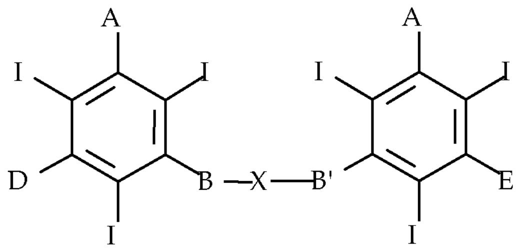

(II)

in which:

A, D and E are as above defined,
B and B' the same or different the one another, represent a covalent bond, or a group selected from the groups of formula -CON(R)-, -N(R)CO- and -N($COR_3$)-, in which:

R is as above said;
$R_3$ is H or a $C_1$-$C_3$ alkyl, optionally substituted by one or more hydroxyl groups; and

X is a $C_1$-$C_6$ alkylene, optionally interrupted by a group selected from -O-, -N- and -S-, and optionally substituted by one or more hydroxyl groups,

with the proviso that in the above formulas (I) and (II) at least one of R is H.

**11.** The CEST-MR procedure of claim 10 wherein the radiographic contrast agent is selected from the group consisting of: Iopamidol, Iodixanol, Iohexol, Ioversol, Iomeprol, Iopromide Iodamide, and possible combinations thereof.

**12.** The CEST-MR procedure of claim 6 to obtain concentration independent CERT-based CEST-MRI images on a human or animal body organ, region, fluid or tissue that comprises:

a) employing a radiographic contrast agent for the exogenous CEST agent in step i) of claim 1;
b) collecting a Z-spectrum and obtaining a Saturation Transfer effect for each of the different flip angles;
c) calculating a ratiometric value of the ST effect as a function of the RF irradiation pulses flip angles, by comparing ST effect values obtained at different RF irradiation pulses flip angles, and
d) obtaining a concentration independent image of said human or animal body organ, region, fluid or tissue.

**13.** The CEST-MR procedure of claim 12 that is carried out *in vitro (ex vivo).*

**14.** The CEST-MR procedure of claim 12 that is carried out *in vivo,* on a human or animal body organ, region, fluid or tissue, said human or animal having been previously administered with the radiographic contrast agent.

**15.** The CEST-MR procedure of claim 4 wherein the calculated ratiometric ST effect is responsive to a physical or chemical parameter of diagnostic interest.

**16.** The CEST-MR procedure of claim 15 for determining the physical or chemical parameter or for obtaining a concentration-independent map of the physical or chemical parameter of diagnostic interest in a human or animal body organ, region, fluid or tissue, that comprises:

A) employing a radiographic contrast agent for the exogenous CEST agent in step i) of claim 1;
B) collecting a Z-spectrum and obtaining a Saturation Transfer effect for each of the different flip angles;
C) calculating a ratiometric value of the ST effect as a function of the RF irradiation pulses flip angles, by a comparative ratio of ST effects obtained at different RF irradiation pulses flip angles acting on the same resonance; and
D) obtaining, from the calculated ratiometric ST effect a measure or a concentration independent map of the physical or chemical parameter of interest.

**17.** The CEST-MR procedure of claim 16 that is carried out *in vitro (ex vivo).*

**18.** The CEST-MR procedure of claim 16 that is carried out *in vivo,* on a human or animal body organ, region, fluid or tissue to which the exogenous CEST agent has been previously administered.

**19.** The CEST-MR procedure of claim 15 performed on a patient to whom a suitable amount of a radiographic contrast agent has been previously administered, that comprises:

i) optionally recording an anatomical MR image of a body organ, region or tissue of interest in said patient;
ii) employing a radiographic contrast agent for the exogenous CEST agent in step i) of claim 1;
iii) acquiring a Z-spectrum,
iv) interpolating the Z-spectrum, and translating the interpolated curve in each image voxel in order to fix the minimum of the curve to zero offset value,
v) calculating the ST effect in the patient body organ, region or tissue of interest at any desired frequency offset,
vi) calculating the ratiometric value of the ST effect for a single resonance, by the comparison of ST effects at different RF flip angles,
vii) obtaining a parametric map of the parameter of interest by using a previously calculated calibration curve; and, optionally,
viii) superimposing the resulting parametric map to the optionally recorded anatomical MR image, in order to spatially localize the region(s) where the parameter of interest is generated.

**20.** The CEST-MR procedure of any one of claims 16-19 wherein the physical or chemical parameter of diagnostic interest is selected from the group consisting of: temperature, pH, partial pressure of oxygen, partial pressure of carbon dioxide, ion or metabolite concentration, and enzymatic activity.

**21.** The CEST-MR procedure of any one of claims 9-20 wherein the radiographic contrast agent is Iodixanol or Iopamidol.

**Patentansprüche**

**1.** CEST-MR-Verfahren, umfassend:

i) Anwenden eines Pulssättigungsschemas durch Bestrahlen einer einzelnes-mobiles-Proton-Frequenz eines exogenen CEST-Mittels in einer Probe mit einem Zug von Radiofrequenz(RF)-Bestrahlungspulsen mit verschiedenen Flipwinkeln und einer konstanten mittleren Leistung $B_1$, definiert als die Quadratwurzel des mittleren quadratischen Bestrahlungsfelds $B_1$ über die gesamte Zeitdauer jedes Pulszugs, zum Induzieren eines Sättigungstransfer-Effekts (ST-Effekt) auf der Basis von Chemical Exchange Rotation Transfer (CERT); und
ii) Erfassen von CEST-Signalen auf CERT-Basis von der Probe.

**2.** CEST-MR-Verfahren gemäß Anspruch 1, wobei das Verfahren auf ratiometrischer Basis steht.

**3.** CEST-MR-Verfahren gemäß Anspruch 2, umfassend Berechnen eines ratiometrischen Werts des ST-Effekts als Funktion der verschiedenen RF-Bestrahlungspuls-Flipwinkel.

**4.** CEST-MR-Verfahren gemäß einem der Ansprüche 2 oder 3, umfassend:

a) Berechnen eines ratiometrischen Werts des ST-Effekts durch Vergleichen von ST-Effekt-Werten, die bei verschiedenen RF-Bestrahlungspuls-Flipwinkeln gemessen sind;
b) Bestimmen eines konzentrationsunabhängigen CEST-Werts.

**5.** CEST-MRI-Verfahren gemäß Anspruch 4, wobei der Schritt a) das Berechnen einer Ratiometrie von gepulste-RF-Flipwinkeln durch Verwendung der Gleichung (3) umfasst,

$$RPA = \frac{\left[(M_0 - M_s)/M_s\right]_{RF_{FA1}}}{\left[(M_0 - M_s)/M_s\right]_{RF_{FA2}}} \qquad (3).$$

6. CEST-MR-Verfahren gemäß Anspruch 4, wobei der konzentrationsunabhängige CEST-Wert verwendet wird, um ein konzentrationsunabhängiges CEST-Bild bereitzustellen.

7. CEST-MR-Verfahren gemäß Anspruch 4, wobei der konzentrationsunabhängige CEST-Wert verwendet wird, um ein konzentrationsunabhängiges parametrisches Bild bereitzustellen.

8. CEST-MR-Verfahren gemäß einem der Ansprüche 1-7, wobei das exogene CEST-Mittel diamagnetisch ist.

9. CEST-MR-Verfahren gemäß Anspruch 8, wobei das diamagnetische Mittel ein radiographisches Kontrastmittel mit wenigstens einem Amid-Proton ist.

10. CEST-MR-Verfahren gemäß Anspruch 9, wobei das radiographische Kontrastmittel ein iodiertes Kontrastmittel der Formel (I),

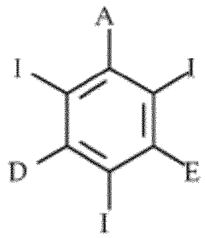

(I)

wobei:

A, D und E gleich oder voneinander verschieden ausgewählt sind aus den Gruppen der Formeln $-CON(R)R_1$,$-COOH$, $-CONH_2$ und - $(CH_2)_{0-1}N(R)-COR_2$; wobei:

R unabhängig voneinander H oder $R_1$ ist;
$R_1$ ein $C_1$-$C_6$-Alkyl, gegebenenfalls substituiert mit einem oder mehreren Hydroxy, $C_1$-$C_5$-Alkoxy, $C_1$-$C_5$-Hydroxyalkoxy oder ein Kohlenhydratrest ist; und
$R_2$ ein $C_1$-$C_6$-Alkyl, gegebenenfalls unterbrochen von einem oder mehreren Hydroxy, $C_1$-$C_5$-Alkoxy oder eine $C_1$-$C_5$-Hydroxyalkoxygruppe ist;

oder der Formel (II) ist,

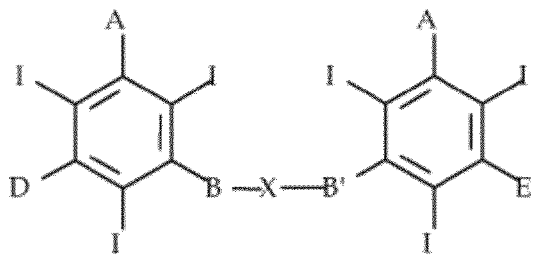

(II)

wobei:

A, D und E wie vorstehend definiert sind,
B und B' gleich oder voneinander verschieden eine kovalente Bindung oder eine Gruppe ausgewählt aus den Gruppen der Formeln $-CON(R)-$, $-N(R)CO-$ und $-N(COR_3)-$ sind, wobei:

R wie vorstehend beschrieben ist;
$R_3$ H oder ein $C_1$-$C_3$-alkyl ist, gegebenenfalls substituiert mit einer oder mehreren Hydroxygruppen; und
X ein $C_1$-$C_6$-Alkylen ist, gegebenenfalls unterbrochen von einer Gruppe ausgewählt aus -0-, -N- und -S- und gegebenenfalls substituiert mit einer oder mehreren Hydroxygruppen,
mit der Maßgabe, dass in den vorstehenden Formeln (I) und (II) wenigstens ein R H ist.

**11.** CEST-MR-Verfahren gemäß Anspruch 10, wobei das radiographische Kontrastmittel ausgewählt ist aus der Gruppe bestehend aus: Iopamidol, Iodixanol, Iohexol, Ioversol, Iomeprol, Iopromid, Iodamid und möglichen Kombinationen davon.

**12.** CEST-MR-Verfahren gemäß Anspruch 6 zum Erhalten von konzentrationsunabhängigen CEST-MRI-Bildern auf CERT-Basis von einem menschlichen oder Tier-Körperorgan, -bereich, -fluid oder -gewebe, umfassend:

a) Verwenden eines radiographischen Kontrastmittels als das exogene CEST-Mittel bei Schritt i) von Anspruch 1;
b) Aufnehmen eines Z-Spektrums und Erhalten eines Sättigungstransfer-Effekts für jeden der verschiedenen Flipwinkel;
c) Berechnen eines ratiometrischen Werts des ST-Effekts als Funktion der RF-Bestrahlungspuls-Flipwinkel durch Vergleichen von ST-Effekt-Werten, die bei verschiedenen RF-Bestrahlungspuls-Flipwinkeln erhalten sind, und
d) Erhalten eines konzentrationsunabhängigen Bilds des menschlichen oder Tier-Körperorgans, -bereichs, -fluids oder -gewebes.

**13.** CEST-MR-Verfahren gemäß Anspruch 12, das *in vitro* (*ex vivo*) durchgeführt wird.

**14.** CEST-MR-Verfahren gemäß Anspruch 12, das *in vivo* an einem menschlichen oder Tier-Körperorgan, -bereich, -fluid oder -gewebe durchgeführt wird, wobei dem Menschen oder Tier zuvor das radiographische Kontrastmittel verabreicht worden ist.

**15.** CEST-MR-Verfahren gemäß Anspruch 4, wobei der berechnete ratiometrische ST-Effekt auf einen physikalischen oder chemischen Parameter von diagnostischem Interesse anspricht.

**16.** CEST-MR-Verfahren gemäß Anspruch 15 zum Bestimmen des physikalischen oder chemischen Parameters oder zum Erhalten einer konzentrationsunabhängigen Karte des physikalischen oder chemischen Parameters von diagnostischem Interesse in einem menschlichen oder Tier-Körperorgan, -bereich, -fluid oder -gewebe, umfassend:

A) Verwenden eines radiographischen Kontrastmittels als das exogene CEST-Mittel bei Schritt i) von Anspruch 1;
B) Aufnehmen eines Z-Spektrums und Erhalten eines Sättigungstransfer-Effekts für jeden der verschiedenen Flipwinkel;
C) Berechnen eines ratiometrischen Werts des ST-Effekts als Funktion der RF-Bestrahlungspuls-Flipwinkel durch ein Vergleichsverhältnis von ST-Effekten, die bei verschiedenen RF-Bestrahlungspuls-Flipwinkeln, die bei der gleichen Resonanz wirken, erhalten sind; und
D) aus dem berechneten ratiometrischen ST-Effekt Erhalten eines Maßes oder einer konzentrationsunabhängigen Karte des physikalischen oder chemischen Parameters von Interesse.

**17.** CEST-MR-Verfahren gemäß Anspruch 16, das *in vitro* (*ex vivo*) durchgeführt wird.

**18.** CEST-MR-Verfahren gemäß Anspruch 16, das *in vivo* an einem menschlichen oder Tier-Körperorgan, -bereich, -fluid oder -gewebe durchgeführt wird, an das/den zuvor das exogene CEST-Mittel verabreicht worden ist.

**19.** CEST-MR-Verfahren gemäß Anspruch 15, durchgeführt an einem Patienten, an den zuvor eine geeignete Menge eines radiographischen Kontrastmittels verabreicht worden ist, umfassend:

i) gegebenenfalls Aufzeichnen eines anatomischen MR-Bilds eines Körperorgans, -bereichs oder -gewebes von Interesse des Patienten;
ii) Verwenden eines radiographischen Kontrastmittels als das exogene CEST-Mittel bei Schritt i) von Anspruch 1;
iii) Aufnehmen eines Z-Spektrums;
iv) Interpolieren des Z-Spektrums und Übersetzen der interpolierten Kurve in jedem Bildvoxel, um das Minimum der Kurve auf einen Verschiebungswert von null festzulegen,

v) Berechnen des ST-Effekts in dem Körperorgan, -bereich oder -gewebe des Patienten von Interesse bei jeder gewünschten Frequenzverschiebung,
vi) Berechnen des ratiometrischen Werts des ST-Effekts für eine einzelne Resonanz durch Vergleichen der ST-Effekte bei verschiedenen RF-Flipwinkeln,
vii) Erhalten einer parametrischen Karte des Parameters von Interesse durch Verwenden einer zuvor berechneten Kalibrierkurve; und gegebenenfalls
viii) Überlagern der erhaltenen parametrischen Karte mit dem gegebenenfalls aufgezeichneten anatomischen MR-Bild, um den/die Bereich(e), an denen der Parameter von Interesse erzeugt wird, räumlich zu lokalisieren.

**20.** CEST-MR-Verfahren gemäß einem der Ansprüche 16-19, wobei der physikalische oder chemische Parameter von diagnostischem Interesse ausgewählt ist aus der Gruppe bestehend aus: Temperatur, pH-Wert, SauerstoffPartialdruck, Kohlendioxid-Partialdruck, Ionen- oder Metabolitenkonzentration und enzymatischer Aktivität.

**21.** CEST-MR-Verfahren gemäß einem der Ansprüche 9-20, wobei das radiographische Kontrastmittel Iodixanol oder Iopamidol ist.

## Revendications

**1.** Procédure de CEST-MR qui comprend :

i) l'application d'un schéma de saturation d'impulsion par irradiation d'une fréquence de proton mobile unique d'un agent de CEST exogène dans un échantillon avec un train d'impulsions d'irradiation de radiofréquence (RF), à différents angles de bascule et à une puissance moyenne constante $B_1$, définie comme la racine carrée du champ d'irradiation $B_1$ carré moyen sur la période entière de chaque train d'impulsion,
pour l'induction d'un effet de transfert de saturation (effet ST) à base de transfert de rotation par échange chimique (CERT) ; et
ii) la détection de signaux CEST à base de CERT dudit échantillon.

**2.** Procédure de CEST-MR selon la revendication 1, ladite procédure étant ratiométrique.

**3.** Procédure de CEST-MR selon la revendication 2 qui comprend le calcul d'une valeur ratiométrique de l'effet ST en fonction des différents angles de bascule des impulsions d'irradiation RF.

**4.** Procédure de CEST-MR selon l'une quelconque des revendications 2 ou 3 qui comprend :

a) le calcul d'une valeur ratiométrique de l'effet ST par comparaison des valeurs d'effet ST mesurées à différents angles de bascule d'impulsions d'irradiation RF ;
b) la détermination d'une valeur de CEST indépendante de la concentration.

**5.** Procédure de CEST-MRI selon la revendication 4, l'étape a) comprenant le calcul d'un ratiométrique d'angles de bascule RF pulsés par exploitation de l'équation (3)

$$RPA = \frac{[(M_0 - M_s)/M_s)]_{RF_{FA1}}}{[(M_0 - M_s)/M_s)]_{RF_{FA2}}} \qquad (3).$$

**6.** Procédure de CEST-MR selon la revendication 4, la valeur de CEST indépendante de la concentration étant utilisée pour fournir une image de CEST indépendante de la concentration.

**7.** Procédure de CEST-MR selon la revendication 4, la valeur de CEST indépendante de la concentration étant utilisée pour fournir une image paramétrique indépendante de la concentration.

**8.** Procédure de CEST-MR selon l'une quelconque des revendications 1 à 7, l'agent de CEST exogène étant diamagnétique.

**9.** Procédure de CEST-MR selon la revendication 8, l'agent diamagnétique étant un agent de contraste radiographique

possédant au moins un proton d'amide.

**10.** Procédure de CEST-MR selon la revendication 9, l'agent de contraste radiographique étant un agent de contraste iodé de formule (I)

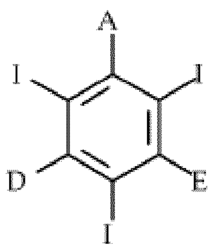

(I)

dans laquelle :

A, D, et E, identiques ou différents les uns des autres, sont choisis parmi les groupes de formule-CON(R)$R_1$, -COOH, -$CONH_2$ et -$(CH_2)_{0-1}$N(R)-$COR_2$ ; dans lesquels :

R, indépendamment l'un de l'autre, est H ou $R_1$ ;
$R_1$ est un $C_{1-6}$-alkyle éventuellement substitué par un ou plusieurs hydroxyles, $C_{1-5}$-alcoxy, $C_{1-5}$-hydroxyalcoxy, ou un radical glucide ; et
$R_2$ est un $C_{1-6}$-alkyle éventuellement interrompu par un ou plusieurs groupes hydroxyle, $C_{1-5}$-alcoxy, ou $C_{1-5}$-hydroxyalcoxy ;

ou de formule (II)

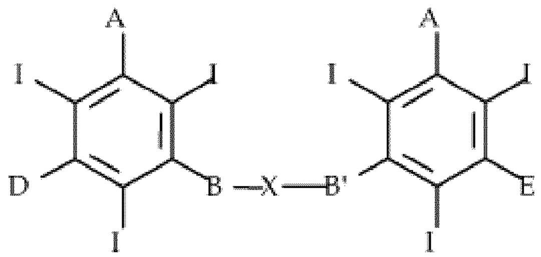

(II)

dans laquelle

A, D et E sont tels que définis ci-dessus,
B et B', identiques ou différents l'un de l'autre, représentent une liaison covalente, ou un groupe choisi parmi les groupes de formule -CON(R)-, -N(R)CO- et-N($COR_3$)-, dans lesquels :

R est tel que mentionné ci-dessus ;
$R_3$ est H ou un $C_{1-3}$-alkyle, éventuellement substitué par un ou plusieurs groupes hydroxyle ; et

X est un $C_{1-6}$-alkylène, éventuellement interrompu par un groupe choisi parmi -0-, -N- et -S-, et éventuellement substitué par un ou plusieurs groupes hydroxyle,
étant entendu que dans les formules ci-dessus (I) et (II) au moins l'un parmi R est H.

**11.** Procédure de CEST-MR selon la revendication 10, l'agent de contraste radiographique étant choisi dans le groupe

constitué par : l'iopamidol, l'iodixanol, l'iohexol, l'ioversol, l'ioméprol, l'iopromide, l'iodamide, et des combinaisons possibles correspondantes.

**12.** Procédure de CEST-MR selon la revendication 6 pour obtenir des images de CEST-MRI à base de CERT indépendantes de la concentration sur un organe, une région, un fluide ou un tissu corporel(le) humain(e) ou animal(e) qui comprend :

a) l'emploi d'un agent de contraste radiographique pour l'agent de CEST exogène dans l'étape i) de la revendication 1 ;
b) le recueil d'un spectre Z et l'obtention d'un effet de transfert de saturation pour chacun des différents angles de bascule ;
c) le calcul d'une valeur ratiométrique de l'effet ST en fonction des angles de bascule des impulsions d'irradiation RF, par comparaison des valeurs d'effet ST obtenues à différents angles de bascule des impulsions d'irradiation RF, et
d) l'obtention d'une image indépendante de la concentration de ladite/dudit organe, région, fluide ou tissu corporel(le) humain(e) ou animal(e).

**13.** Procédure de CEST-MR selon la revendication 12 qui est mise en œuvre *in vitro* (*ex vivo*).

**14.** Procédure de CEST-MR selon la revendication 12 qui est mise en œuvre *in vivo,* sur un organe, une région, un fluide ou un tissu corporel (le) humain(e) ou animal(e), l'agent de contraste radiographique ayant été administré précédemment audit humain ou audit animal.

**15.** Procédure de CEST-MR selon la revendication 4, l'effet ST ratiométrique calculé étant sensible à un paramètre physique ou chimique d'intérêt diagnostique.

**16.** Procédure de CEST-MR selon la revendication 15 pour la détermination du paramètre physique ou chimique ou pour l'obtention d'une cartographie indépendante de la concentration du paramètre physique ou chimique d'intérêt diagnostique dans un organe, une région, un fluide ou un tissu corporel(le) humain(e) ou animal(e), qui comprend :

A) l'emploi d'un agent de contraste radiographique pour l'agent de CEST exogène dans l'étape i) de la revendication 1 ;
B) le recueil d'un spectre Z et l'obtention d'un effet de transfert de saturation pour chacun des différents angles de bascule ;
C) le calcul d'une valeur ratiométrique de l'effet ST en fonction des angles de bascule des impulsions d'irradiation RF, par un rapport comparatif d'effets ST obtenus à différents angles de bascule des impulsions d'irradiation RF agissant sur la même résonance ; et
D) l'obtention, à partir de l'effet ST ratiométrique calculé, d'une mesure ou d'une cartographie indépendante de la concentration du paramètre physique ou chimique d'intérêt.

**17.** Procédure de CEST-MR selon la revendication 16 qui est mise en œuvre *in vitro* (*ex vivo*).

**18.** Procédure de CEST-MR selon la revendication 16 qui est mise en œuvre *in vivo,* sur un organe, une région, un fluide ou un tissu corporel (le) humain(e) ou animal(e) auquel/à laquelle l'agent de CEST exogène a été administré précédemment.

**19.** Procédure de CEST-MR selon la revendication 15 réalisée sur un patient auquel on a administré précédemment une quantité appropriée d'un agent de contraste radiographique, qui comprend :

i) éventuellement l'enregistrement d'une image de MR anatomique d'un organe, d'une région ou d'un tissu corporel(le) d'intérêt dans ledit patient ;
ii) l'emploi d'un agent de contraste radiographique pour l'agent de CEST exogène dans l'étape i) de la revendication 1 ;
iii) l'acquisition d'un spectre Z,
iv) l'interpolation du spectre Z, et la translation de la courbe interpolée dans chaque voxel d'images afin de fixer le minimum de la courbe à une valeur de décalage de zéro,
v) le calcul de l'effet ST dans l'organe, la région ou le tissu corporel(le) d'intérêt du patient à un quelconque décalage de fréquence souhaité,

vi) le calcul de la valeur ratiométrique de l'effet ST pour une unique résonance, par la comparaison des effets ST à différents angles de bascule RF,

vii) l'obtention d'une cartographie paramétrique du paramètre d'intérêt à l'aide d'une courbe de calibration précédemment calculée ; et, éventuellement,

viii) la superposition de la cartographie paramétrique résultante sur l'image de MR anatomique éventuellement enregistrée, afin de localiser dans l'espace la ou les région(s) où le paramètre d'intérêt est généré.

**20.** Procédure de CEST-MR selon l'une quelconque des revendications 16 à 19, le paramètre physique ou chimique d'intérêt diagnostique étant choisi dans le groupe constitué par : la température, le pH, la pression partielle d'oxygène, la pression partielle de dioxyde de carbone, la concentration d'un ion ou d'un métabolite, et une activité enzymatique.

**21.** Procédure de CEST-MR selon l'une quelconque des revendications 9 à 20, l'agent de contraste radiographique étant l'iodixanol ou l'iopamidol.

Figure 1

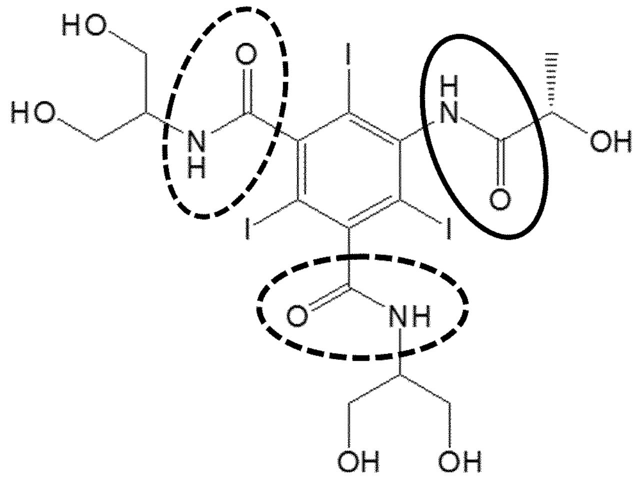

**Iopamidol**

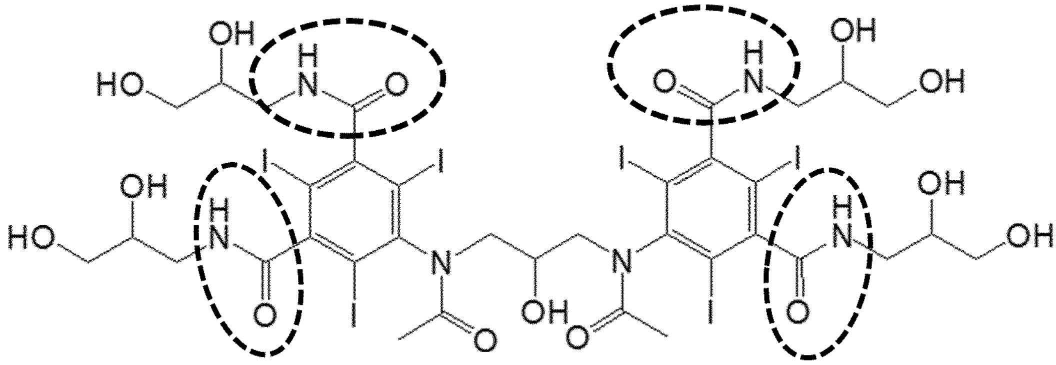

**Iodixanol**

Figura 2

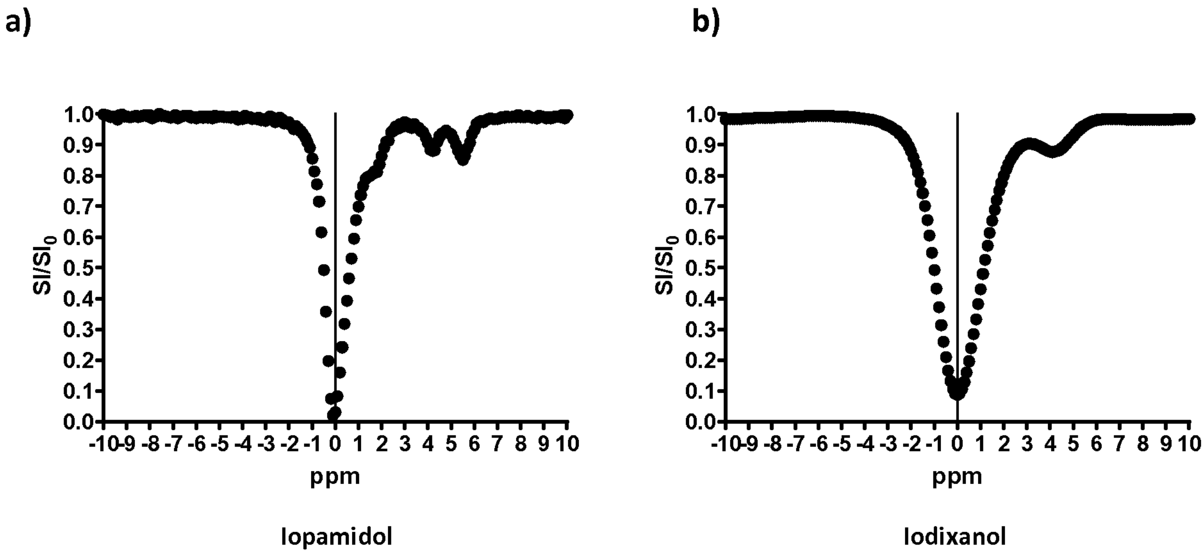


Figure 3

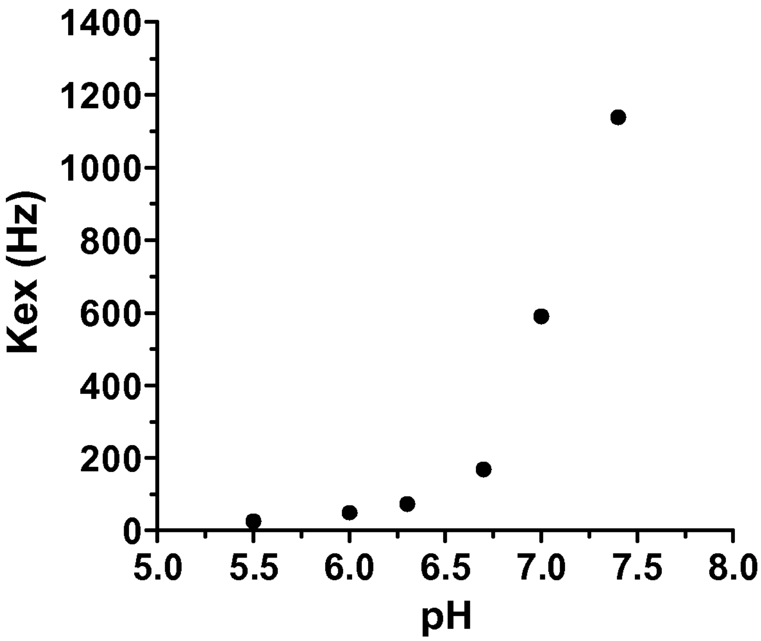

Figure 4

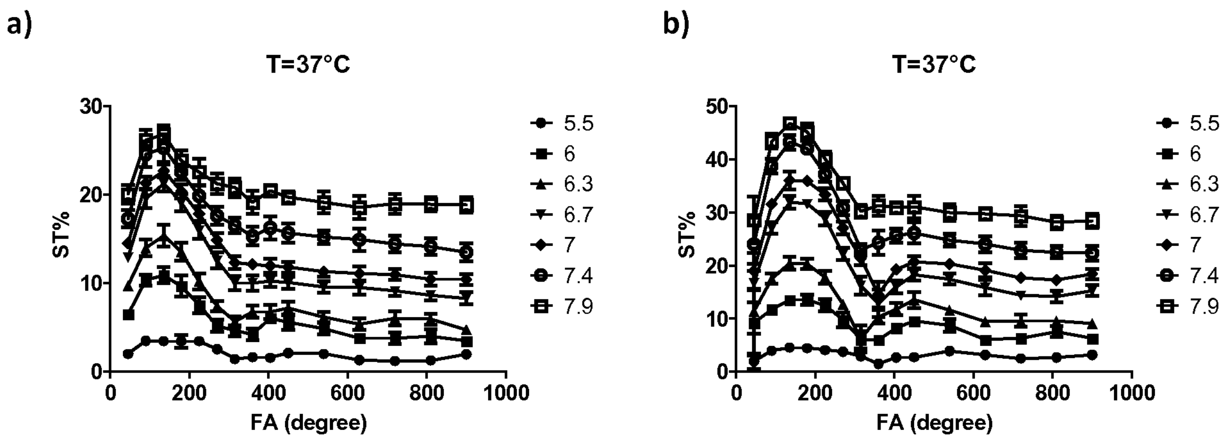
a)
T=37°C
ST%
FA (degree)
5.5
6
6.3
6.7
7
7.4
7.9
b)
T=37°C
ST%
FA (degree)
5.5
6
6.3
6.7
7
7.4
7.9

Figure 5

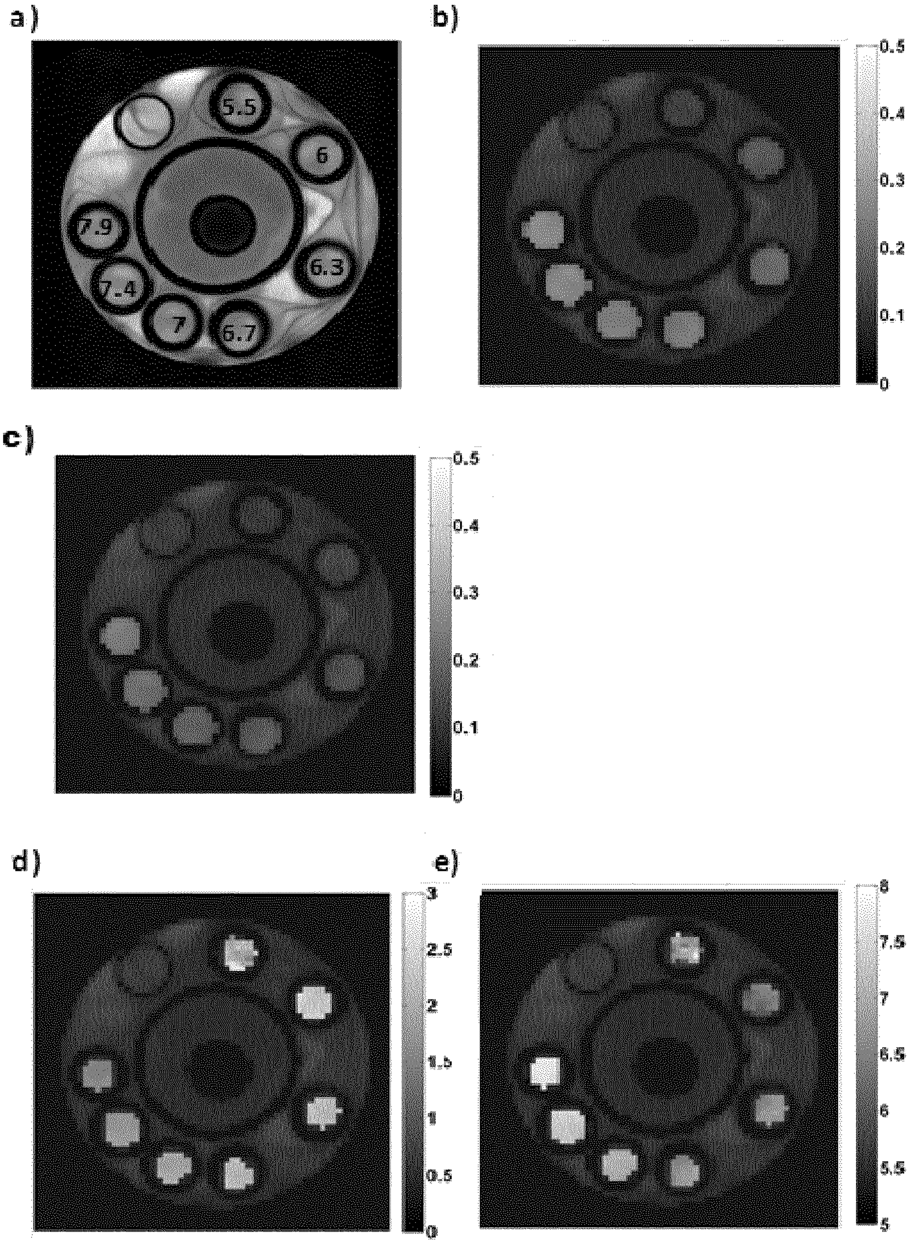
a)
5.5
6
7.9
6.3
7.4
7
6.7
b)
0.5
0.4
0.3
0.2
0.1
0
c)
0.5
0.4
0.3
0.2
0.1
0
d)
3
2.5
2
1
0.5
0
e)
8
7.5
7
6.5
6
5.5
5

Figure 6

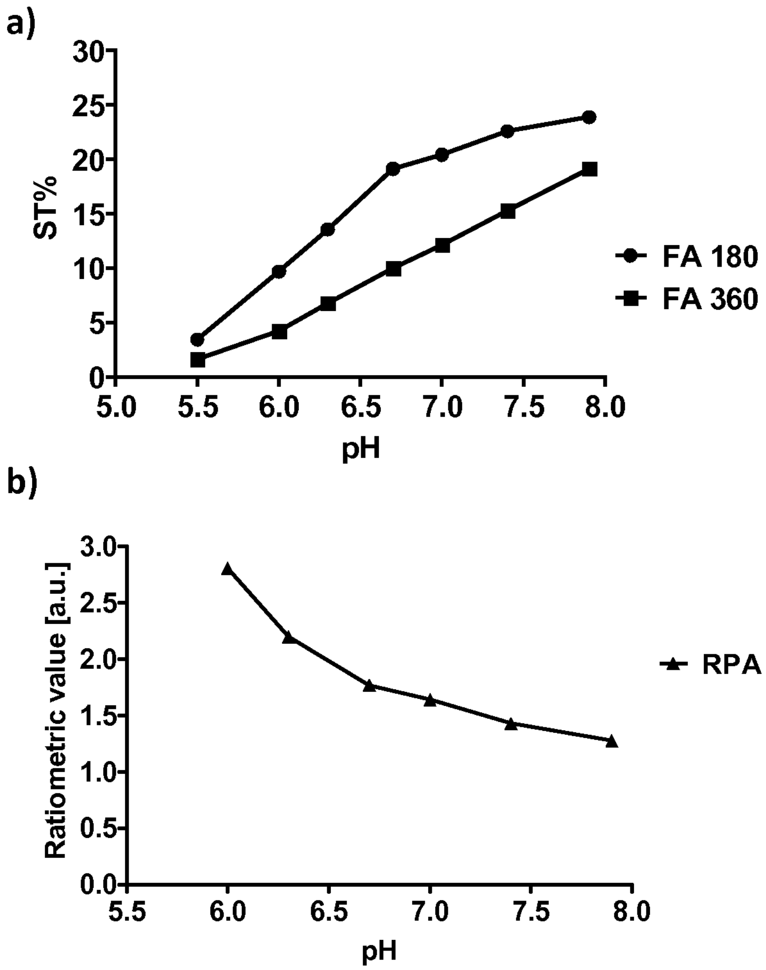
a)
30
25
20
15
10
5
0
ST%
5.0
5.5
6.0
6.5
7.0
7.5
8.0
pH
FA 180
FA 360
b)
3.0
2.5
2.0
1.5
1.0
0.5
0.0
Ratiometric value [a.u.]
5.5
6.0
6.5
7.0
7.5
8.0
pH
RPA

Figure 7

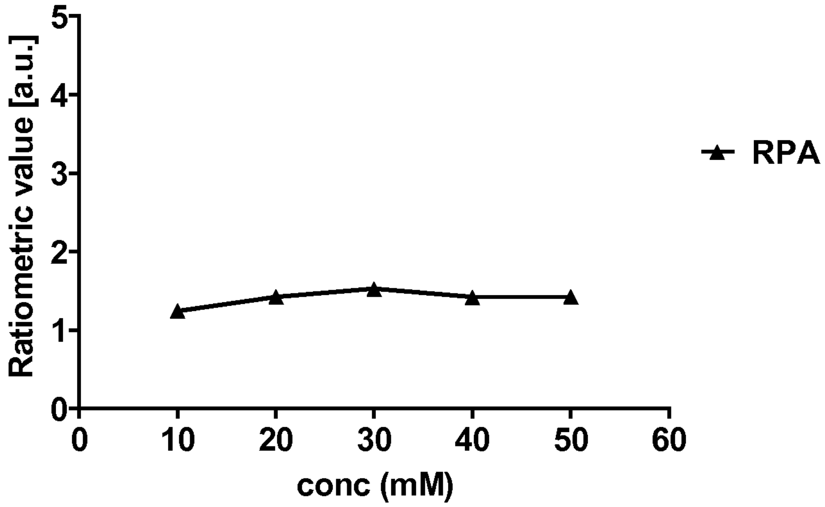


Figure 8

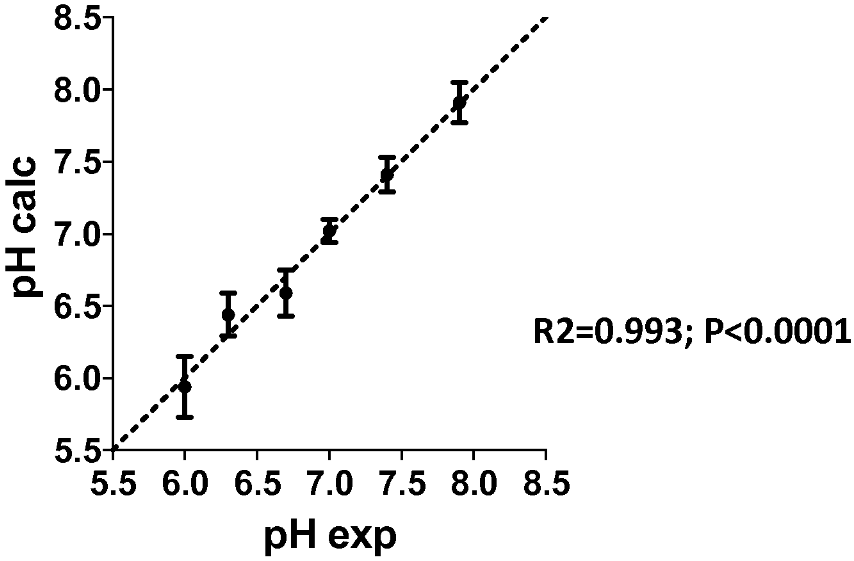

Figure 9

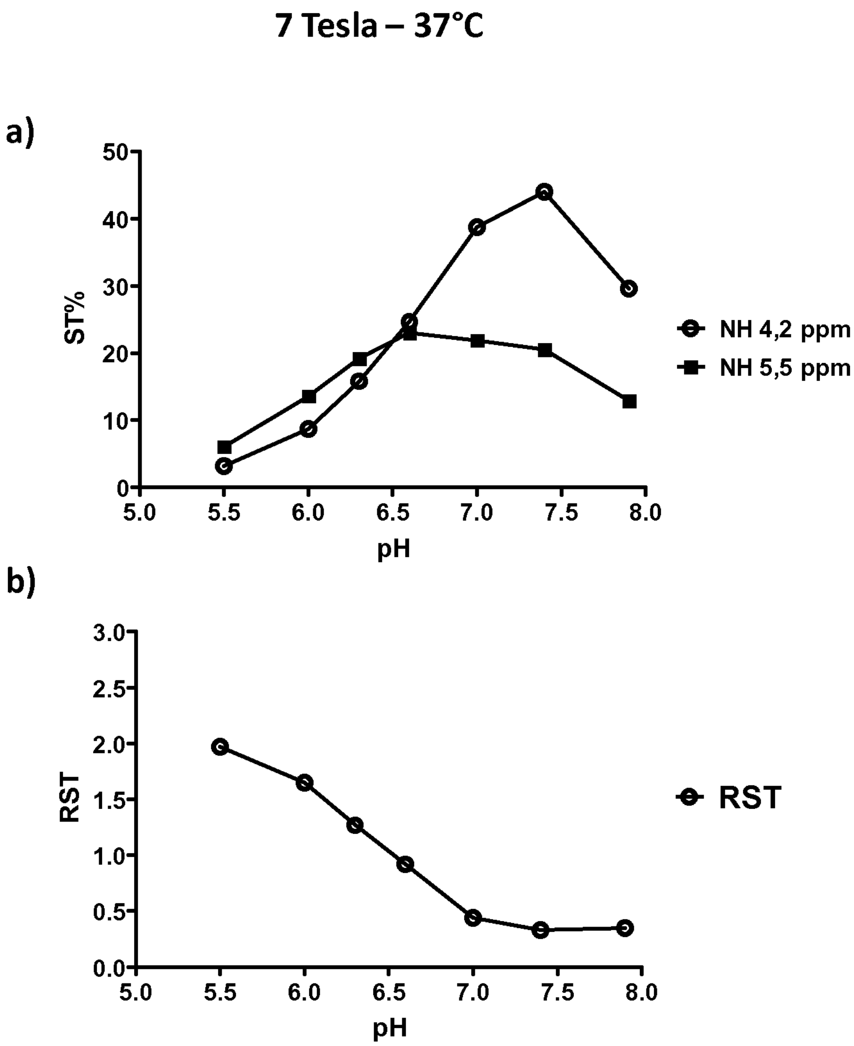
7 Tesla – 37°C
a)
ST%
50
40
30
20
10
0
5.0
5.5
6.0
6.5
7.0
7.5
8.0
pH
NH 4,2 ppm
NH 5,5 ppm
b)
RST
3.0
2.5
2.0
1.5
1.0
0.5
0.0
5.0
5.5
6.0
6.5
7.0
7.5
8.0
pH
RST

Figure 10

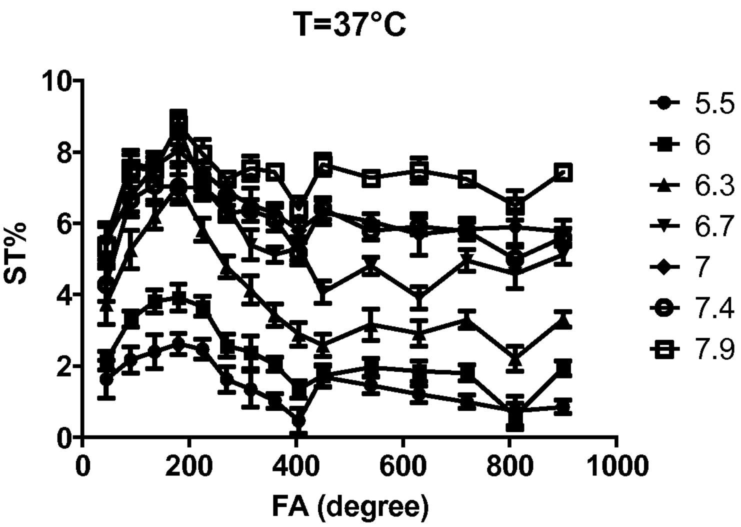


Figure 11

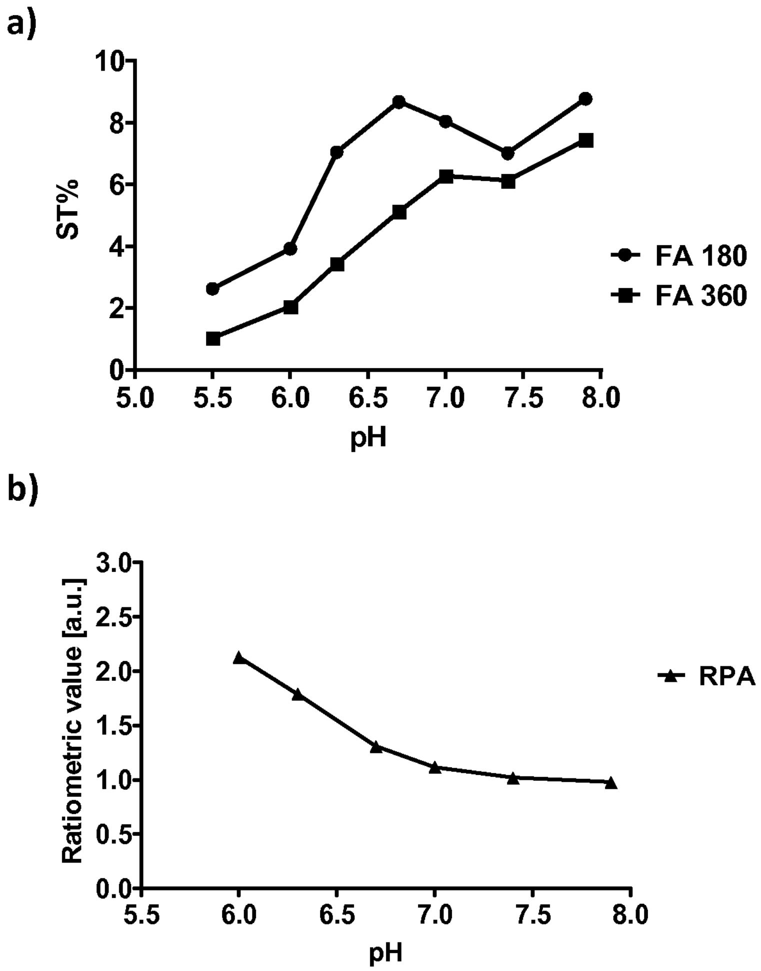

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0066180 A
- US 8211404 B


### Non-patent literature cited in the description


- **ENZO TERRENO ; DANIELA DELLI CASTELLI ; SILVIO AIME.** Encoding the frequency dependence in MRI contrast media: the emerging class of CEST agents. *Contrast Media Mol. Imaging,* 2010, vol. 5, 78-98
- *J. Am. Chem. Soc,* 2001, vol. 123, 1517-1518
- *Magnetic Resonance Imaging. Chem. Rev.,* 2010, vol. 110, 2960-3018
- *Magn. Reson. Med.,* 2000, vol. 44, 799-802
- *J. Am. Chem. Soc.,* 2014, vol. 136, 14333
- *Magn Reson Med,* 2008, vol. 60, 834
- *Magn Reson Med,* 2011, vol. 65, 1620
- *Magn Reson Med,* 2011, vol. 66, 1100-1108
- *Reson Med,* 2012, vol. 68, 711-719
- *Magn Reson Med,* 2011, vol. 66, 1100
- *Magn Reson Med,* 2011, vol. 65, 202
- *Magn. Reson. Med.,* 2005, vol. 53, 830-834
- *Magn Reson Med,* 2013, vol. 70, 859
- Development and validation of a smoothing-splines-based correction method for improving the analysis of CEST-MR images. *CONTRAST MEDIA & MOLECULAR IMAGING,* 2008, vol. 3 (4), 136-149
- Methods for an improved detection of the MRI-CEST effect. *CONTRAST MEDIA & MOLECULAR IMAGING,* 2009, vol. 4 (5), 237-247
- *Contrast Media Mol Imaging,* 2009, vol. 4, 237-47
- *Magn. Reson Med,* 2012, vol. 68, 711-719
- *CONTRAST MEDIA & MOLECULAR IMAGING,* 2008, vol. 3 (4), 136-149
- *CONTRAST MEDIA & MOLECULAR IMAGING,* 2009, vol. 4 (5), 237-247
- **T. L. HWANG ; A. J. SHAKA.** Water Suppression That Works. Excitation Sculpting Using Arbitrary Wave-Forms and Pulsed-Field Gradients. *J. Magn. Reson. Ser. A,* 1995, vol. 112 (3), 275-279
- *Investigative Radiology,* 12 October 2015, vol. 00, 00-00